# EUROPEAN PATENT APPLICATION

(11) **EP 4 811 385 A1**
(43) Date of publication of application: **23.09.2026**
(21) Application number: 25165213.7
(22) Date of filing: 21.03.2025
(51) Int. Cl.: G16H 30/40, A61C 13/00, G16H 50/50

(54) **METHOD AND SYSTEM FOR AUTOMATICALLY DESIGNING A DENTAL RESTORATION**

(71) Applicant: 3Shape A/S, 1060 Copenhagen K (DK)
(72) Inventor: ASK, Erik, 1060 Copenhagen K (DK); ÖJELUND, Henrik, 1060 Copenhagen K (DK); HANSEN, Peter Steen, 1060 Copenhagen K (DK)
(74) Representative: Zacco Denmark A/S

(57) **Abstract**

Disclosed is a computer-implemented method for automatically designing a dental restoration for placing in a dentition of a patient, the method comprising the steps of: obtaining digital three-dimensional (3D) surface data representing a surface of the dentition of the patient in 3D space, wherein the surface comprises a preparation surface configured to receive the dental restoration; pre-processing the 3D surface data to generate a discretized 3D model comprising granularity elements defining a discretized representation of at least a part of the 3D surface data, wherein the at least a part of the 3D surface data comprises the preparation surface; pre-processing the discretized 3D model to generate an encoded representation of the discretized 3D model, wherein the encoded representation comprises the granularity elements, and each granularity element is encoded with at least distance information from the point to at least one functional surface; processing the encoded representation by using a trained model configured to process the encoded representation; receiving an output of the processing from the trained model, wherein the output comprises at least a part of the dental restoration; and automatically designing the dental restoration based on the received output.

## Description

### Technical field

The present invention generally relates to a method and a system for automatically designing a dental restoration for placing in a dentition of a patient. More particularly, the invention relates to a computer-implemented method for designing the dental restoration by using a trained neural network.

### Background

Restorative dentistry is a branch of dentistry which focuses on diagnosing and treating diseases of the teeth and of the supporting structures of the teeth of a patient. The primary goal of restorative dentistry is to rehabilitate both aesthetic and function of the patient's dentition, through the restoration of damaged and/or missing parts of the teeth. Restorative dental procedures may include fillings, inlays, onlays, crowns, bridges, implants and dentures, and they share the common goal to ensure that the patient may chew, speak and smile properly after successfully completing the restorative procedure.

A first focus of restorative dentistry is to design a dental restoration, also commonly referred to as dental prosthesis, which resembles realistic features of the damaged or missing part(s) of the patient's dentition. In preparing the patient's dentition to receive the dental prosthesis, a dental practitioner may modify the dentition by removing damaged and/or decayed tooth material by means of abrasive rotating dental instruments. The result of the preparation process is a tooth surface which is prepared for receiving the dental prosthesis, i.e. a so-called preparation surface, whose boundary of the preparation surface is often referred to as a margin line or preparation line. If a good fit between the dental restoration and the preparation surface is not achieved when mounting the restoration on the prepared tooth, there is a risk that a gap may develop between the preparation surface and the dental prosthesis. As bacteria may develop in said gap and cause further damage of the tooth, it is crucial to properly design the dental restoration in such a way that a good fit is achieved.

Nowadays, digital dentistry enables dental practitioners to acquire intraoral scan data of the patient's dentition by means of an intraoral scanning device. The intraoral scan data may be used to generate a three-dimensional (3D) digital model of the dentition, which may comprise digital information regarding the preparation surface and the surrounding dentition. The dental restoration may then be digitally designed using computer aided design (CAD) and manufactured through computer aided manufacturing (CAM) or additive manufacturing (3D printing) techniques.

**In** recent years, neural networks have spread widely in digital dentistry as a supporting tool to design the missing tooth parts and surfaces. Neural networks enable to build a system which is capable of learning from a large number of exemplary data, and to make new predictions based on this learning. Neural networks having different underlying structures or architectures are possible, wherein the neural network structure influences both the type of data used for training the neural network and the kind of data required as input of the network. Examples of neural network structures used in the context of digital dentistry include, but are not limited to, PointNet and Encoding Graph Structures. Independently on the format of the input data, the trained neural network may output a prediction of the shape of the dental restoration.

Despite the progress achieved in the use of neural networks in digital dentistry, currently used neural network architectures suffer from several limitations. One problem with existing solutions is that they are not inherently suitable to handle the generation of new points or nodes, e.g. points or nodes describing the missing parts of a tooth. While the structure of the input data, i.e. the existing dentition and preparation surface, is known and well determined, the structure of the output data, i.e. the shape of the missing surface, is largely unknown and it may not share a one-to-one correspondence with the input data. Therefore, existing neural network architectures may not be suitable to predict the shape of missing parts or surfaces of the dentition.

Another limitation of existing neural network structures is that they are not inherently suitable for designing multiple and/or interconnected dental restorations, e.g. for the design of a dental bridge. Such a situation may require generating multiple intermediate outputs, i.e. one output for each part of the restoration to be designed, and then combining these multiple intermediate outputs to design the final dental restoration. However, to date there is not an effective way to combine the outputs of commonly used neural network structures while preserving the inherent structure of each output, e.g. the relationship between the nodes of the graph in the case of an Encoding Graph Structure or between the points of the point cloud in the case of a PointNet structure. While such an output combination may be possible, it requires to process the intermediate outputs before merging them. The processing may lead to a loss of information and resolution, besides increasing memory consumption and computational time. Therefore, currently used neural network structures are not suitable for designing composite dental restorations such as dental bridges.

Given the importance of designing a functional and aesthetically acceptable dental restoration, it is crucial to develop a neural network which can maximize the likelihood of generating dental restorations with precise and realistic anatomical shapes and functions. A neural network able to deal with dynamic and unknown output structures may also be crucial to design partial restorations, such as veneers or inlays, and/or composite restorations, such as dental bridges.

### Summary

It is one aspect of the present disclosure to provide a computer-implemented method that overcomes the above-mentioned disadvantages. In particular, in one aspect there is disclosed herein is a computer-implemented method for automatically designing a dental restoration for placing in a dentition of a patient, the method comprising the steps of:
a. obtaining digital three-dimensional (3D) surface data representing a surface of the dentition of the patient in 3Dspace, wherein the surface comprises a preparation surface configured to receive the dental restoration;
b. pre-processing the 3D surface data to generate a discretized 3D model comprising granularity elements defining a discretized representation of at least a part of the 3D surface data, wherein the at least a part of the 3D surface data comprises the preparation surface;
c. pre-processing the discretized 3D model to generate an encoded representation of the discretized 3D model, wherein the encoded representation comprises the granularity elements, and each granularity element is encoded with at least distance information from the point to at least one functional surface;
d. processing the encoded representation by using a trained model configured to process the encoded representation;
e. receiving an output of the processing from the trained model, wherein the output comprises at least a part of the dental restoration; and
f. automatically designing the dental restoration based on the received output.

The discretized representation comprises granularity elements, i.e. finite elements, collectively describing at least a geometry of the at least a part of the 3D surface data. Advantageously, discretizing the at least a part of the 3D surface data may reduce the computational complexity of the design process, since any processing unit of available computer systems is configured to process discretized models. Furthermore, the pre-processing of the discretized 3D model may be intended as a local processing of each granularity element comprised in the discretized 3D model, which results in an encoded representation. This ensures that each granularity element in the encoded representation is accurately located in 3D space relative to the same at least one functional surface. In other words, the encoded representation encompasses information about whether each granularity element is located inside the functional surface, outside the functional surface or on the boundary of the functional surface. The at least one functional surface may be a surface which influences the functional properties of the dentition and of the dental restoration to be designed. The encoded representation is advantageous for processing comprising typical Boolean operations, such as union and intersection, as these operations can be easily performed by comparing and combining distance information. This is particularly advantageous when combining multiple geometrical shapes. Therefore, the present disclosure provides an efficient and computationally advantageous method for automatically designing dental restorations, in particular partial dental restorations and composite dental restorations.

The 3D surface data may comprise data representing a surface of an upper jaw of the dentition and data representing a surface of a lower jaw of the dentition. In one example, the 3D surface may be acquired by scanning the surface of the upper jaw and the surface of the lower jaw by means of an intraoral scanning device. In another example, a gypsum model of the upper jaw and lower jaw is obtained and then scanned by means of a scanning device configured to acquire digital surface information of the gypsum models. This ensures that digital information relative to at least teeth and gingiva of the patient's can be obtained and used for designing an aesthetically and functional dental restoration.

The method may further comprise obtaining 3D surface data of the upper jaw in occlusion with the lower jaw, thereby obtaining information of the relative alignment between the upper jaw and the lower jaw. 3D surface data of the upper jaw in occlusion with the lower jaw can be obtained by scanning a bite of the patient by means of an intraoral scanning device, or by scanning a gypsum model of the bite of the patient by means of a scanning device. Advantageously, the relative alignment between the upper jaw and the lower jaw enables to allocate the upper and lower to a common reference frame. This ensures that the dental restoration can be designed in such a way that the patient can chew properly after mounting the dental restoration in the patient's dentition.

The granularity elements of the discretized 3D model may comprise a plurality of facets of a mesh. The mesh may be a polygonal mesh, such as a triangular mesh. Therefore, each of the plurality of facets may be a triangle in the case where the mesh is a triangle mesh, or another polygon in the case where the mesh is a polygonal mesh. Advantageously, facets comprise information about a local curvature of a 3D surface, and they therefore provide a discretized representation suitable for describing a complex geometry such as the surface of the dentition.

The granularity elements of the discretized 3D model may comprise a point cloud, wherein the point cloud comprises a plurality of points in 3D space. Advantageously, point clouds are suitable for geometric computations, e.g. distance computation, and they further provide an effective discretized representation of irregular surfaces, such as ridges and/or grooves of teeth.

The computer-implemented method may further comprise determining or retrieving one or more structural features of the preparation surface, wherein the one or more structural features comprise one or more of a location of the preparation surface, and/or a margin line of the preparation surface. Determining the location of the preparation surface enables also to determine the properties of the surrounding teeth of the preparation surface, which are crucial to design a functional and aesthetically pleasing dental restoration. For example, the dental restoration may be designed with a color which resembles the color of teeth surrounding the location of the dental restoration. Further, the location of the dental restoration influences a maximum size to be enforced when designing the dental restoration to ensure that the restoration properly fits in the patient's dentition. Determining the margin line is crucial to design a dental restoration which, once mounted in the patient's mouth, does not leave any gap between the restoration and the preparation surface. This is important to prevent the development of bacteria which may cause further damage to the dentition.

The method may further comprise determining or retrieving one or more design constraints to be enforced when designing the dental restoration, wherein the one or more design constraints comprise one or more of an insertion direction of the dental restoration, a planned manufacturing material of the dental restoration and/or an inner surface of the dental restoration. The above mentioned one or more design constraints may be automatically determined, e.g. by means of one or more algorithms configured to determine each of the one or more design constraint based on the obtained 3D surface data of the dentition. Alternatively, each of the one or more design constraints may be manually determined, e.g. by a dental practitioner, and stored as metadata or as augmentation data associated with the 3D surface data. The metadata and/or augmentation data may be automatically retrieved when designing the dental restoration. Properly determining or retrieving the insertion direction of the dental restoration ensures that a dental restoration properly fitting onto the preparation surface may be designed, therefore avoiding the development of gaps which may lead to microleakage, caries or debonding of the final dental restoration. The planned manufacturing material influences the mechanical properties of the dental restoration to be designed, such as its mechanical strength and durability. The inner surface of the dental restoration directly interfaces the prepared tooth structure once the restoration is mounted in the patient's dentition, hence determining or retrieving the inner surface ensures mechanical retention and/or chemical bonding with dental cement or adhesives. Therefore, determining or retrieving the one or more design constraints is important to design a dental restoration which is both aesthetically acceptable and functional.

A minimal thickness of the dental restoration to be enforced when designing the dental restoration may be determined based on the planned manufacturing material of the dental restoration. As mentioned above, the planned manufacturing material influences the mechanical properties of the dental restoration, such as its strength and durability. **In** particular, the dental restoration may be enforced to have a minimal thickness, corresponding to the minimal thickness required by the planned manufacturing material to resist to external forces, e.g. the force applied by other teeth in the patient's dentition when the patient chews.

An input of the trained model may further comprise one or more of the relative alignment between the upper jaw and lower jaw, the one or more structural features of the preparation surface, and/or the one or more design constraints to be enforced when designing the dental restoration. Advantageously, the relative alignment between the upper jaw and lower jaw, the one or more structural features of the preparation surface and/or the one or more design constraints may be used by the trained model as constraints to be enforced in generating the output. This ensures that the output comprising the at least a part of the dental restoration may be used directly, i.e. without additional fine-tuning and/or post-processing, in the automated design of the dental restoration. As the output of the trained model may already meet the functional requirements imposed by the input of the trained model, the computational time needed to automatically design the dental restoration may be reduced.

The discretized model may comprise a discretization grid. Advantageously, the discretization grid comprises finite elements, i.e. the granularity elements, which may be suitable for processing by trained models used by the method, e.g. trained neural networks. In particular, the discretization grid ensures that the at least a part of the 3D surface data is simplified, which may reduce the computational load of the method and make it faster to process large datasets by means of the trained model.

The discretization grid may be a structured discretization grid encompassing at least the preparation surface and having a desired size. Therefore, the granularity elements are arranged in a regular and predictable pattern, namely the relationship between neighboring granularity elements is well defined. As there is no need to store connectivity information explicitly, the structured discretization grid enables both to save memory and to optimize the computational time of the method. This speeds up the design process of the dental restoration, which is an advantage both for the dental practitioner and for the patient for whom the dental restoration is designed. Advantageously, the desired size of the discretization grid ensures that the output of the method may achieve a desired computational accuracy. However, in some cases the discretization grid may be an unstructured discretization grid, which may be used to describe complex surface geometries.

The position of the preparation surface within the structured discretization grid may be based on a pose of the preparation surface. The pose of the preparation surface may be intended as a local coordinate system for the preparation surface, where the local coordinate system comprises a preparation surface specific origin and three mutually-orthogonal axes intersecting at the origin. The pose of the preparation surface may define a reference frame, e.g. a Cartesian reference frame, in which the dental restoration may be designed. Therefore, defining the position of the preparation surface within the discretization grid ensures that the preparation surface is accurately located in space in the discretized 3D model. This ensures that the dental restoration may be designed in a well-defined geometrical space.

The desired size of the structured discretization grid may be based on a determined or retrieved pose of the preparation surface. This ensures that the structured discretization grid is aligned with the patient's natural tooth, i.e. the tooth to restore, such that the dental restoration may be designed in a reference system aligned with the surrounding teeth. In this way, a functional dental restoration which properly fits in the patient's dentition can be designed.

The pose of the preparation surface may be determined based on the obtained 3D surface data. This ensures that the pose is determined in an automated manner, e.g. by means of one or more algorithms configured to determine the pose, improving the accuracy of the design process of the dental restoration. For example, the pose of the preparation surface may be determined by using one or more mathematical algorithms such as Principal Component Analysis (PCA).

In some embodiments, the pose of the preparation surface may be retrieved based on a received input. For example, the pose of the preparation surface may be manually determined by the user and stored as metadata and/or augmentation data associated with the 3D surface data. The metadata and/or augmentation data may be fed into the trained model as an input, i.e. the received input may comprise the metadata and/or augmentation data. Retrieving the pose of the preparation surface may reduce the computational time of the method, since it does not require to run an algorithm to computationally determine the pose.

The desired size of the structured discretization grid may be based on a desired resolution of the output of the trained model. Advantageously, this ensures that the dental restoration can be designed with a desired level of details.

The structured discretization grid may entirely encompass the obtained 3D surface data, whereby the discretized model is a discretized representation of the obtained 3D surface data. Therefore, the bite of the patient's dentition may be taken into account when designing the dental restoration, enabling to design the dental restoration with enhanced accuracy.

The desired size of the structured discretization grid may be a predefined feature, or the desired size of the structured discretization grid may be selected by a user. Advantageously, setting the size of the structured discretization grid as a predefined feature ensures that the size may be pre-optimized for the method, reducing the variability and errors which may be caused by inconsistent selection of the size of the grid. On the other hand, manually selecting the size of the discretization grid may enable the user to customize the level of details of the dental restoration to be designed, thereby enhancing the flexibility of the method.

The structured discretization grid may comprise an x-axis, a y-axis and a z-axis. The x-axis may be oriented along a tooth-line, the y-axis may be oriented along a lingual or buccal dimension, and the z-axis may be oriented along an occlusal direction. The tooth line may be intended as a tangent line to a dental arch, wherein the dental arch intersects the tooth for which the restoration is designed. Therefore, the dental restoration may be designed in a reference system aligned with the natural dentition of the patient. This ensures that, once designed and manufactured, the dental restoration properly fits in the patient's dentition, enabling the patient to properly chew, smile and talk.

Pre-processing the discretized 3D model may comprise determining, for each granularity element of the discretized 3D model, at least one distance value, wherein the at least one distance value quantifies a distance of the granularity element from the at least one functional surface. Pre-processing the discretized 3D model may further comprise encoding each granularity element of the discretized 3D model with the corresponding at least one distance value to generate the encoded representation. This ensures that each granularity element is unambiguously located in space relative to the at least one functional surface, providing a simplified representation of a 3D geometry of the patient's dentition described by the intraoral scan data. Therefore, generating the encoded representation is advantageous for reducing the computational overhead of the method and enhancing scalability.

The design of the dental restoration will be typically influenced by more than one functional surface. For example, the design of a functional restoration may take into account both the neighboring natural teeth and the teeth in the opposite jaw to ensure a proper bite relationship between the upper jaw and the lower jaw. In other words, each granularity element may be encoded with a distance value of the granularity element from each of one or more functional surfaces, which may be advantageous to capturing complex and realistic features of the patient's dentition.

The at least one functional surface may comprise one or more of a surface of a preparation jaw of the dental restoration, a surface of an antagonist jaw of the dental restoration, and/or a minimal surface boundary. In order to design a functional dental restoration, e.g. a lasting dental restoration which allows the patient to chew and bite properly, the space available for the dental restoration between neighboring natural teeth and the distance to the opposite jaw should be taken into account. Furthermore, the minimal surface boundary defines the minimal outer surface that the dental restoration should have in order not to break. Therefore, encoding the discretized 3D model with the at least distance information of each granularity element to at least one of the aforementioned functional surfaces ensures that the resulting encoding representation comprises information enabling to design a functional restoration.

The computer-implemented method may further comprise determining a planned manufacturing material for use in a manufacturing process of the dental restoration, and determining the minimal surface boundary based on the planned manufacturing material. Importantly, the planned manufacturing material influences the minimal thickness needed for the dental restoration not to break, and the outer boundary of the minimal thickness defines the minimal outer surface boundary. Therefore, the minimal surface boundary defines the minimal outer surface required for designing a lasting and robust dental restoration.

The minimal surface boundary may define a design constraint to be enforced when designing the dental restoration. The design constraint may comprise a minimal outer surface required to design the dental restoration with one or more planned functional properties. Advantageously, the enforced design constraint ensures that a functional dental restoration may be designed.

The one or more planned functional properties may comprise a mechanical strength. Thus, a durable and long-lasting dental restoration may be automatically designed.

The encoded representation may be a distance field representation. A distance field may be intended as a representation where at each point within the field the distance from that point to the closest point on any surface within the domain, e.g. within the discretized 3D model and/or the dentition of the patient, is known. Therefore, the distance field representation provides an implicit representation of the 3D geometry described by the 3D surface data, which may enable to manipulate the data without the need for detailed surface geometry. This may significantly save both computational time and memory needed to design the dental restoration.

The trained model may be a trained neural network. Trained neural networks can learn from training data sets and adapt to new data, which makes them flexible and suitable tools for predicting unknown shapes, e.g. the shape of the dental restoration to design.

The trained neural network may be a Deep Convolutional Neural Network. Advantageously, Deep Convolutional Neural Networks are suitable to extract relevant information from the input data and to generate an accurate output at low computational cost. Their usage may therefore speed up the design process of the dental restoration and enhance the accuracy of the output of the process.

A resolution of the input of the trained model may be the same as a resolution of the output of the trained model. Advantageously, the resolution of the output, i.e. the level of detail of the predicted dental restoration, can be controlled by changing the resolution of the input, namely the resolution of the encoded representation. This may also enable to adjust the computational time of the design process based on the desired level of detail of the dental restoration to be designed. For example, temporary dental restorations may be designed with less details than permanent ones.

The dental restoration may comprise one or more of a crown, a veneer, an inlay, an onlay, a dental bridge, a denture, and/or a removable partial denture. These are among the most commonly needed dental restorations. However, the present disclosure is not limited to the above-mentioned list of dental restorations, and any other dental restoration type may be designed according to the present disclosure.

The output of the trained model may comprise a distance field representing at least a part of an outer surface of the dental restoration. Distance fields are suitable to be manipulated through Boolean operations, e.g. multiple distance fields can be easily combined to obtain complex geometrical shapes. Advantageously, this makes the method of the present disclosure suitable for designing multiple dental restorations which can then be merged into a single dental restoration, such as a dental bridge. Furthermore, the method of the present disclosure may be suitable to design partial dental restorations, such as an inlays, since the output of the trained model may be merged with a distance field representing a 3D geometry of an intact part of the tooth for which the dental restoration is designed.

Automatically designing the dental restoration may comprise converting the output distance field to a mesh model. The mesh model provides an explicit representation of the geometry encoded by the distance field, which may be suitable to manufacture the designed dental restoration. Furthermore, the mesh model may be rendered, e.g. in a display of a computer system on which the method is executed, and manually manipulated to adjust the level of detail of the designed dental restoration.

Automatically designing the dental restoration may further comprise retrieving a digital template of a restorative tooth and morphing the output of the trained neural network using the digital template of the restorative tooth. Thus, the level of detail of the output may be refined to obtain a more aesthetically accurate and/or more functional dental restoration.

The morphing may comprise adapting the digital template to the output of the trained model to refine one or more anatomical features of the dental restoration, the one or more anatomical features comprising ridges and grooves. Thus, once manufactured and mounted in the patient's dentition, the dental restoration may enable the patient to efficiently grind and crush food during chewing. A proper design of the grooves may be beneficial also for enabling speech articulation of the patient.

The dental restoration may be a partial dental restoration. This is particularly advantageous when only a part of a tooth surface is damaged and/or missing, whereas the main tooth structure is intact.

The partial restoration may comprise a veneer or an inlay. Thus, the method of the present disclosure enables to restore the aesthetic of the patient's dentition, in the case of veneer, and to restore cavities which have not progressed into more extensive tooth decay, such as the cavities caused by dental caries.

The method may further comprise combining the output of the trained model with at least a part of the surface comprised in the obtained 3D surface data to automatically design the partial restoration. This may enable to adjust the output of the trained model based on its fit onto the part of the surface comprised in the obtained 3D surface data. Furthermore, the result of the combination between the output and the existing 3D surface data may be used to design one or more additional dental restorations, such as multiple dental restorations for the same dentition may be designed according to the method disclosed herein.

The at least a part of the surface may comprise a tooth surface which is intact after a preparation process of the tooth, wherein the tooth is configured to receive the dental restoration. Advantageously, the output of the trained model may be refined to resemble more closely the properties of the remaining part of the prepared teeth, and it may be further refined to improve the fit between the designed dental restoration and the prepared teeth.

The at least a part of the surface may comprise a tooth surface before the tooth suffered of any damage, wherein the tooth is configured to receive the dental restoration. Advantageously, the output of the trained model may be refined to resemble more closely the properties of the tooth before the tooth got damaged, which may enable to design a more realistic dental restoration.

The method may further comprise determining a certainty score of the output of the trained model. Advantageously, the certainty score quantifies the accuracy and reliability of the output of the trained model. Thus, said output may be adjusted and refined either automatically or manually, based on the certainty score.

The method may further comprise iteratively repeating any of one or more of the method steps described above at least a number *N* of times to design a number *N* of dental restorations. Therefore, multiple dental restorations may be designed for the same patient's dentition by using the same 3D surface data, which is advantageous both for the dental practitioner and for the patient in need of the dental restoration.

The at least a number N of times may be based on a convergence criterion. This enables to minimize the numerical errors which may occur in the iterative method, enhancing the accuracy of the output.

The number N of dental restorations may be connected to form a dental bridge. This is particularly advantageous when the patient is missing one or more neighboring teeth.

The output may comprise an outer surface of the dental restoration. Thus, the external functional and aesthetic properties of the dental restoration may be automatically obtained by the trained model and used to automatically design the dental restoration.

The output may comprise an inner structure of the dental restoration. It is an advantage that a prediction of the whole dental restoration design may be automatically obtained by the trained model and used to automatically design the dental restoration.

**In** another aspect, there is disclosed herein a program product comprising instructions which, when executed by a computer, cause the computer to perform the method steps described herein. Thus, in practice the method disclosed herein can be executed by any computer system configured to read the computer program product disclosed herein. This is an advantage, as the instructions comprised in said computer program ensure that the execution of one or more steps of the method disclosed herein is automated.

**In** yet another aspect, there is disclosed herein a non-volatile computer-readable medium comprising instructions which, when executed by a computer, cause the computer to perform the method according to any of the disclosed embodiments. Thus, in practice the instructions to perform the method disclosed herein can be performed on any computer system configured to read the non-volatile computer-readable medium disclosed herein. This is an advantage, as said instructions cab be retained also when the computer system is turned off, enabling the user to access the instructions and/or the data stored on the non-volatile computer-readable medium at any time and from any computer system configured to read the non-volatile computer-readable medium disclosed herein.

**In** yet another aspect, there is disclosed herein a system which may comprise an intraoral scanner. The system may further comprise a computer system. Said computer system may comprise a display, a communication interface, a processing unit and a memory unit. The memory unit may contain a program context executable by the processing unit, the program content comprising executable instructions to:
g. obtain 3D surface data of the dentition of the patient representing a surface of the dentition in three-dimensional (3D) space, wherein the surface comprises a preparation surface configured to receive the dental restoration;
h. pre-process the obtained 3D surface data to generate a discretized 3D model comprising granularity elements defining a discretized representation of at least a part of the 3D surface data, wherein the at least a part of the 3D surface data comprises the preparation surface;
i. pre-process the discretized 3D model to generate an encoded representation of the discretized 3D model, wherein the encoded representation comprises the granularity elements, and each granularity element is encoded with at least distance information from the point to at least one functional surface;
j. process the encoded representation by using a trained model configured to process the encoded representation;
k. receive an output of the processing from the trained model, wherein the output comprises at least a part of the dental restoration; and
l. automatically design the dental restoration based on the received output.

Advantageously, the intraoral scanner may enable the dental practitioner to perform a scan of the dentition of the patient, and therefore to acquire the digital 3D surface data relative to the dentition. It is a further advantage that the obtained 3D surface data of the dentition of the patient are first pre-processed to generate a discretized representation of at least a part of the 3D surface data. The discretized representation comprises granularity elements collectively describing at least a geometry of the at least a part of the scan data in 3D space. Advantageously, discretizing the at least a part of the 3D surface data may reduce the computational complexity of the design process, since the processing unit is configured to process discretized models. Furthermore, the pre-processing of the discretized 3D model is a local processing of each granularity element of the discretized 3D model, which results in an encoded representation. Each granularity element of the encoded representation is encoded with local information comprising at least distance information of the point to at least one functional surface. This ensures that each granularity element in the encoded representation is accurately located in 3D space relative to the same at least one functional surface. Such an encoded representation is advantageous for processing comprising typical Boolean operations, such as union and intersection, as these operations can be easily performed by the processing unit(s) of the computer system disclosed herein by comparing and combining distance values. This is particularly advantageous when predicting geometrical shapes. In particular, the at least a part of the dental restoration can be predicted by inputting the encoded representation in the trained model. Therefore, the present disclosure provides an efficient and computationally advantageous system for automatically designing the dental restoration.

The communication interface may be configured to enable the computer system to exchange data with one or more external devices and with one or more external networks. Thus, the communication interface enables the computer system to send and/or receive data from the intraoral scanner comprised in the system disclosed herein. This is an advantage, as the computer system may receive from the intraoral scanner 3D surface data relative to the patient's dentition. Furthermore, the communication interface enables the computer system to receive and/or send data to an external network which may be configured to perform at least a part of the processing of the intraoral scan data, thereby facilitating and speeding up the design process of the dental restoration.

The one or more external networks may comprise at least one cloud network. Thus, a part of the processing of the 3D surface data may be performed by the at least one cloud network. Advantageously, the cloud network may be configured to handle heavy workloads efficiently, thereby reducing the computational load of the computer system and optimizing the design process of the dental restoration.

The at least one cloud network may comprise executable instructions to process the encoded representation using a trained model. Thus, the computer system may send the generated encoded representation of the discretized 3D model to the cloud network, and receive the output of the trained model of the cloud network by means of the communication interface, without the need of having said trained model stored on the memory unit comprised in the computer system. This significantly saves memory space of the computer system, and it also speeds up the processing of the intraoral scan data to design the dental restoration.

### Brief description of the drawings

Aspects of the present disclosure may be best understood from the following detailed description alongside the following drawings. The drawings are schematic and simplified for the sake of clarity, and they are intended to show details to improve the understanding of the claims, whereas other details may be left out. Throughout the detailed description and the drawings, the same reference numbers are used for identical or corresponding parts. The individual features of each aspect may each be combined with any or all features of other aspects. These and other aspects, features and/or technical effects will be apparent from and elucidated with reference to the illustrations described hereinafter in which:
Figure 1 illustrates a flowchart according to an embodiment of the computer-implemented method disclosed herein;
Figures 2a, 2b and 2c illustrate a schematic presentation of digital 3D surface data of a patient's dentition comprising a preparation surface;
Figure 3 illustrates an example of a schematic workflow to determine a discretization grid according to the present disclosure;
Figures 4a, 4b and 4c illustrate a schematic example of a workflow for designing a dental restoration according to the computer-implemented method of the present disclosure.
Figure 5 illustrates a schematic example of a part of a workflow for designing a partial dental restoration according to the present disclosure.
Figures 6a,6b, 6c and 6d illustrate a flowchart of a schematic example of a workflow to design multiple dental restorations according to the present disclosure.
Figure 7 illustrates a flowchart of a schematic example of a method for training a model according to the present disclosure.
Figure 8 schematically illustrates a system according to the present disclosure.

### Detailed description

The detailed description set forth below in connection with the appended drawings is intended as a description of various examples according to the disclosure. The detailed description includes details for the purpose of providing a thorough understanding of various concepts and examples covered throughout the description. However, it will be apparent to those skilled in the art that these concepts and examples may be practiced without the specific details mentioned or in combination with one or more examples described herein. Several examples of the devices, systems, mediums, programs and methods are described by various modules, components, steps, processes, algorithms, etc. Depending upon particular application, design constraints or other reasons, these elements may be implemented using electronic hardware, computer program, or any combination thereof. **In** the following, several examples of the method and system described herein will be disclosed in more detail.

The solutions presented herein generally aim at improving the automatic design of dental restorations, including crowns, veneers, onlays, inlays, or any other relevant restorative element aiming at restoring and/or improving the functionality and/or the aesthetic of the dental site.

The dentition may comprise a group of dental features of a subject's, i.e. patient's, mouth, such as a group of teeth and their encircling gingiva. The dentition may include any one or more of: tooth/teeth, gingiva, implant(s), dental restoration(s), dental prostheses, edentulous ridge(s), and/or combinations thereof. Alternatively, the dentition may be a gypsum model or a plastic model representing a subject's teeth. As an example, the dentition may comprise teeth and/or gingiva of a subject. The dentition may be only a part of the subject's teeth and/or oral cavity, since the entire set of teeth of the subject is not necessarily scanned during a scanning session. A scanning session may be understood herein as a period of time during which data (such as 2D images) of the dental object is acquired/obtained.

To obtain digital 3D surface data, an intraoral scanner for acquiring images within an intraoral cavity of a patient may be used. Alternatively, a gypsum model of the patient's dentition may be acquired during a clinical visit of the patient to the dental studio. Then, the gypsum model may be scanned by means of a scanning device, such as a laboratory scanning device, to acquire digital 3D surface data relative to the patient's dentition. The intraoral scanner is a handheld intraoral scanner, i.e. a device configured to be held with a human hand. The intraoral scanner comprises one or more projector unit(s) configured for illuminating the dental object with probe light. The intraoral scanner comprises one or more camera unit(s) configured for acquiring 2D images of the dentition. The intraoral scanner may employ any suitable scanning principle such as triangulation-based scanning, stereo vision, structure from motion, confocal scanning, focus scanning, time-of-flight scanning, or other scanning principles. **In** some embodiments, the intraoral scanner employs a triangulation-based scanning principle. As an example, a projector unit and one or more camera units may be utilized to determine points in 3D space based on triangulation. **In** other embodiments, the intraoral scanner employs a focus-based scanning principle. An intraoral scanner using focus scanning technique is further described in EP 2 442 720 B1 by the same applicant.

A projector unit may be understood herein as a device configured for projecting light onto a surface, such as the surface of a dentition. In preferred embodiments, the projector unit is configured to project a pattern of light onto the surface of a dentition. The projector unit may be configured to project a pattern of light such that the pattern of light is in focus at a predefined focus distance measured along a projector optical axis. Features in the pattern of light may be used to extract information on the 3D geometry from the 2D images, e.g. by finding correspondence between a camera pixel in one camera unit observing a particular pattern feature and a projector pixel in the projector unit that generated that particular pattern feature unit or a camera pixel in another camera unit also observing that particular pattern feature, whereby triangulation may be performed.

Each projector unit may comprise one or more light sources. The light source(s) may be configured to generate light of a single wavelength (monochromatic), a distribution, e.g. a Poisson distribution, of wavelengths, or a combination of wavelengths (polychromatic). The combination of wavelengths may be produced by a light source configured to produce light comprising different wavelengths (such as white light). A light source of the one or more light sources may be configured to generate light at other wavelengths than at least some of the other light sources of the one or more light sources, e.g. the one or more light sources may comprise a light source generating red light, a light source generating blue light, and a light source generating green light. In some embodiments, each projector unit comprises a light source for generating white light. Alternatively, the projector unit may comprise multiple light sources such as LEDs individually producing light of different wavelengths (such as red, green, and blue) that may be combined to form light comprising different wavelengths. Thus, the light produced by the light source(s) may be defined by a wavelength defining a specific color, or a range of different wavelengths defining a combination of colors such as white light. In some embodiments, the light source is a diode, such as a white light diode, or a laser diode.

In some embodiments, the intraoral scanner comprises a light source configured for exciting fluorescent material to obtain fluorescence data from the dentition such as from teeth. Such a light source may be configured to produce a narrow range of wavelengths. In other embodiments, the intraoral scanner comprises an infrared light source, which is configured to generate wavelengths in the infrared range, such as between 700 nm and 1.5 µm. In some embodiments, the intraoral scanner comprises one or more light sources selected from the group of: Infrared (IR) light source, near-infrared (NIR) light source, blue light source, violet light source, ultraviolet (UV) light source, and/or combinations thereof. In some embodiments, the intraoral scanner comprises a first light source forming part of the projector unit, and one or more second light sources, e.g. IR-LED(s) and/or UV-LED(s), located in a distal part of the intraoral scanner, such as in the tip of the intraoral scanner.

A camera unit may be understood herein as a device for capturing a 2D image of the dental object. Each camera unit may comprise an image sensor for generating an image based on incoming light e.g. received from the illuminated dental object. The image sensor may comprise a 2-dimensional array of camera pixels. Each camera pixel may comprise one or more subpixels configured to capture light of a certain wavelength or wavelength interval, whereby a camera pixel may capture color data by adding the values of at least some of its associated subpixels. One or more of the subpixels may be configured for capturing light outside the visible spectrum, e.g. UV and/or IR wavelengths.

As an example, the image sensor may be an electronic image sensor such as a charge-coupled device (CCD) or an active-pixel sensor (CMOS sensor). Each image sensor may define an image plane, which may be understood as the plane that contains the object's projected image. Each image obtained by the image sensor(s) may comprise a plurality of image features, wherein each image feature originates from a pattern feature of the projected pattern. In some embodiments, one or more of the camera units comprise a light field camera. Preferably, each camera unit defines a camera optical axis. The camera units may further comprise one or more focus lenses for focusing light.

In some embodiments, the image sensor is a monochrome image sensor, wherein each pixel is associated with a single color channel, e.g. is a grayscale color channel, wherein the value of each pixel represents only an amount of light. In other embodiments, the image sensor is a color image sensor or an image sensor comprising a color filter array on the array of pixels. As an example, the color filter array may be a Bayer filter employing an arrangement of four color filters: Red (R), Green (G), Green (G), and Blue (B). The Bayer filter may also be referred to as an RGGB filter. When utilizing the image sensor data, color pixels may be combined to monochrome pixels of 2 × 2 color pixels for 3D depth reconstruction to obtain the 3D surface data. In this case, the resolution of the 3D depth reconstruction is only half the resolution of the image sensor in each direction. When obtaining texture (color) images the full native resolution is preferably utilized (with color filtered pixels).

The image sensor may have an image frame rate of at least 30 frames per second, such as at least 60 frames per second, or even at least 90 frames per second. At least some of the frames may be dedicated to obtaining primary scan data. In frames dedicated to obtaining primary scan data, the one or more projector units may be configured for probe light comprising one or more discrete wavelengths of light, e.g. by light generated from one or more laser light sources. At least some of the frames may be dedicated to obtaining secondary scan data. At least some of the frames dedicated to obtaining secondary scan data may be dedicated to obtain fluorescence data. The one or more projector units may be configured for emitting UV light during frames dedicated to obtain fluorescence data. At least some of the frames dedicated to obtaining secondary scan data may be dedicated to obtain IR data. The one or more projector units may be configured for emitting IR light during frames dedicated to obtain fluorescence data. At least some of the frames dedicated to obtaining secondary scan data may be dedicated to obtain texture/color data. The one or more projector units may be configured for emitting white light during frames dedicated to obtain fluorescence data. Alternatively, the one or more projector units may be configured for emitting monochromatic light of a first wavelength, e.g. green, during some of the frames dedicated to obtain fluorescence data, and for emitting monochromatic light of a second wavelength, e.g. blue, during some of the frames dedicated to obtain fluorescence data, and for emitting monochromatic light of a third wavelength, e.g. red, during some of the frames dedicated to obtain fluorescence data, whereby the color(s) of the dentition may be calculated by adding the frames dedicated to obtain fluorescence data.

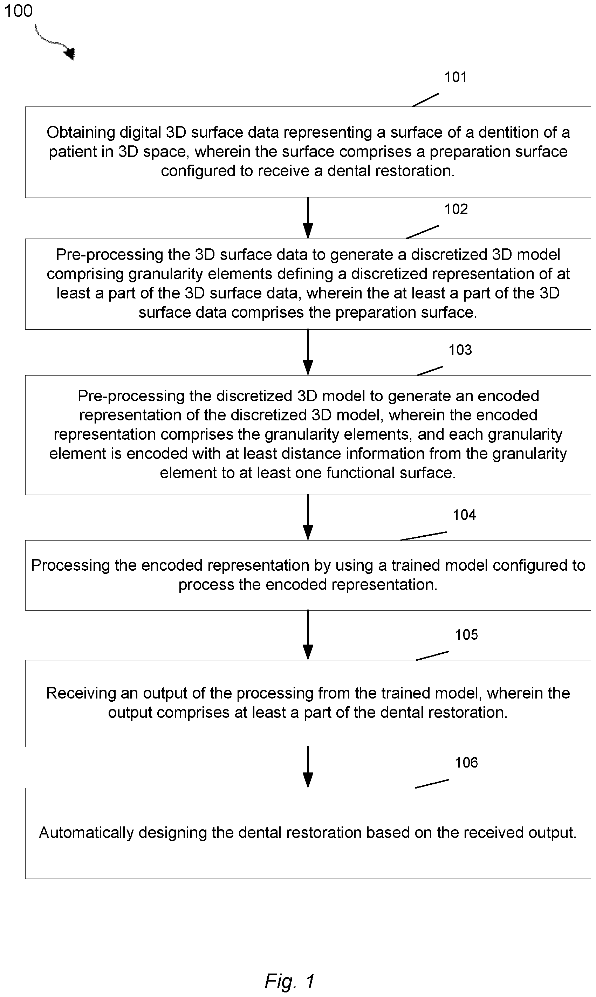

Figure 1 illustrates a flowchart 100 according to a schematic example of the computer-implemented method disclosed herein. In step 101, digital three-dimensional (3D) surface data of a dentition of a patient is obtained. The digital 3D surface data represents a surface of the dentition of the patient in three-dimensional (3D) space, and it comprises a preparation surface which is configured to receive a dental restoration. The dentition is a part of an oral cavity of the patient, and it comprises at least a part of the patient's teeth and gingiva, but it may also comprise soft palate, hard palate and any other part of the patient's oral cavity. The 3D surface data may be acquired during a clinical visit of the patient, for example upon scanning the patient's dentition with an intraoral scanner. Alternatively, a physical impression of the patient's dentition may be obtained during the clinical visit of the patient, i.e. a gypsum impression. The physical impression may then be scanned using a scanning device to generate digital 3D surface data of the gypsum impression, i.e. the digital 3D surface data of the patient's dentition. In some cases, the digital 3D surface data of the dentition may have been acquired during a previous clinical visit of the patient and stored, e.g. on a memory unit, during said previous clinical visit. In this case, the digital 3D surface data may be obtained upon loading the stored digital 3D surface data, e.g. from the memory unit whereon the data has been stored during the previous clinical visit.

The digital 3D surface data may comprise any type of data suitable to describe a surface in 3D space. For example, the 3D surface data may comprise a point cloud, a polygon mesh, a voxel model, a triangulated point cloud, or any other method of storing digital information about a surface of the dentition in 3D space, i.e. a 3D geometry of the dentition. In another example, the 3D surface data may comprise a triangulated mesh, wherein the triangulated mesh comprises a set of triangles which are connected by their common edges or vertices. In general, the 3D surface data represent at least the 3D geometry of the surface of the patient's dentition.

In step 102, the obtained 3D surface data is pre-processed to generate a discretized 3D model comprising granularity elements defining a discretized representation of at least a part of the obtained 3D surface data. The part of the 3D surface data which is discretized comprises the preparation surface. The discretized 3D model may comprise a discretization grid comprising a plurality of granularity elements which collectively represent the discretized version of the at least a part of the 3D surface data. For example, the granularity elements may be points in 3D space, such as points identified by (x, y, z) coordinates. In another example, the granularity elements may be facets of a mesh, such as the triangles of a triangular mesh, or the granularity elements may be the vertices of a mesh, such as the vertices of a triangular mesh. In general, the granularity elements may comprise any finite element suitable for discretizing the 3D geometry of the surface of the dentition. For example, if the 3D surface data comprises a plurality of voxels, in the discretization process the voxels may be divided into finite elements having smaller size than the original voxels, wherein the size of the finite elements may be suitable for further processing, e.g. by means of a trained model. The granularity elements may be herein referred to as "grid points". The discretization grid may be a structured discretization grid, wherein the arrangement of the grid points follows a regular pattern. For example, the density of the grid points in the discretization grid may decrease with a distance from the preparation surface, such that more grid points will be present in a proximity of the preparation surface than at the edges of the discretization grid. In this way, detailed information about the geometry of the preparation surface may be encompassed in the discretization grid. In another example, the grid points may be arranged in a uniform pattern, e.g. the grid points may be uniformly spaced in the discretization grid. In general, the connectivity of the grid points of the structured discretization grid may be implicitly defined by a logical indexing system, such as a Cartesian coordinate system or a curvilinear coordinate system.

In step 103, the discretized 3D model generated in step 102 is pre-processed to generate an encoded representation of the discretized 3D model. The encoded representation comprises the granularity elements of the discretized 3D model, and each granularity element is encoded with at least distance information from the granularity element to at least one functional surface. The functional surface may be any of one or more surfaces influencing one or more functional properties of the dentition. Since the functionality of the dentition should be preserved after the dental restoration is designed, manufactured and mounted in the dentition, it is important to take into account the distance of each granularity element from the functional surface when designing the dental restoration. The one or more functional properties of the dentition may comprise the bite of the patient, the speech and phonetics of the patient, but also the protection and self-cleansing abilities of the patient's dentition. In some examples, the at least one functional surface may comprise any of one or more of a surface of a preparation jaw, the corresponding antagonist jaw, and/or the minimal surface boundary. The latter may be intended as the minimum outer surface that the dental restoration is required to have to ensure its durability and mechanical strength. To guarantee that the dental restoration is designed in such a way that the functional properties of the patient's dentition are preserved, a distance of each grid point to the at least one functional surface may be determined, and each grid point may be encoded with the corresponding distance information. Therefore, each grid point is located in space with respect to the at least one functional surface. In some examples, the distance may be intended as the minimal Euclidean distance between the grid point and the at least one functional surface, i.e. the Euclidean distance from the grid point to the closest point on the at least one functional surface. In another example, the distance may be intended as the minimal color distance between the grid point and the at least one functional surface, i.e. the color distance from the grid point to the closest point on the at least one functional surface. The encoded representation may be a distance field representation, such that the distance of each grid point from the at least one functional surface may be a positive distance value, a negative distance value, or equal to zero. In this example, the at least one functional surface may be intended as an outer surface defining the boundary of an object in the dentition, e.g. the boundary of the preparation jaw, or the boundary of the antagonist surface, or boundary of the minimal boundary surface. If a grid point is encoded with a positive distance value from the at least one functional surface, then the grid point is located outside the shape defined by the at least one functional surface. If a grid point is encoded with a negative distance value from the at least one functional surface, then the grid point is located inside the shape defined by the at least one functional surface. If a point is encoded with a zero-distance value from the at least one functional surface, then the grid point is located on the at least one functional surface, i.e. the grid point is located on the boundary of the shape defined by the at least one functional surface. For example, a grid point encoded with a distance value of -0.3 mm from the antagonist jaw, a distance value of +10 mm from the preparation jaw, and a distance value of +20 mm from the minimal surface boundary is a point belonging to a tooth of the antagonist jaw. In other words, the encoded representation represents an implicit representation of the 3D geometry of at least a part of the dentition. In another example, the encoded representation is a binary representation, wherein each grid point is encoded with a "+" sign or "-" sign depending on whether the grid point is located outside or inside the at least one functional surface, respectively.

In step 104, the encoded representation generated in step 103 is processed by using a trained model configured to process the encoded representation. The trained model may be a pre-trained model that has been trained using a training data set. The training data set may comprise a plurality of completed dental restoration cases. Each completed restoration case may comprise 3D surface data comprising a preparation surface configured to receive a dental restoration, and the corresponding digitally designed dental restoration. For example, the digitally designed dental restorations may have been designed, based on the 3D surface data, by a dental practitioner. Each of the plurality of dental restoration cases may be further augmented with information about a location of a preparation jaw, a location of an antagonist jaw, a pose of the dental restoration, a margin line, a minimal surface boundary, and any other parameter or any other information relevant for designing the dental restoration. A detailed description of methods to train the model according to the present disclosure will be provided later in the detailed description. The trained model may be a neural network, for example the neural network may be a Deep Convolutional Neural Network.

In step 105, an output of the processing is received from the trained model, wherein the output comprises at least a part of the dental restoration. For example, the output may comprise an outer surface of the dental restoration, or the output may comprise the outer surface and an inner structure of the dental restoration. The output received from the trained model may be in the same form as the input fed into the trained model. In other words, if the trained model is fed with an encoded representation in step 104, then the trained model may output an encoded representation of the at least a part of the dental restoration. For example, if the encoded representation used as an input for the trained model is a distance field representation of the discretized 3D model, the output of the trained model may comprise a distance field representing at least a part of the dental restoration.

In step 106, the dental restoration may be automatically designed based on the received output. For example, the received output may be a distance field describing at least a part of the dental restoration. Therefore, digitally designing the dental restoration may comprise converting the output into a mesh model which may be suitable for displaying the output to a user, e.g. on a display of a computer system executing the computer-implemented method 100. In some examples, the received output may be merged with an existing template tooth model, e.g. a digital template which is uploaded from a digital dental library. Accordingly, automatically designing the dental restoration may comprise refining the details of the output based on the merging of the output with the template tooth model. For example, refining the details may comprise refining one or more anatomical features such as ridges and/or grooves of the dental restoration.

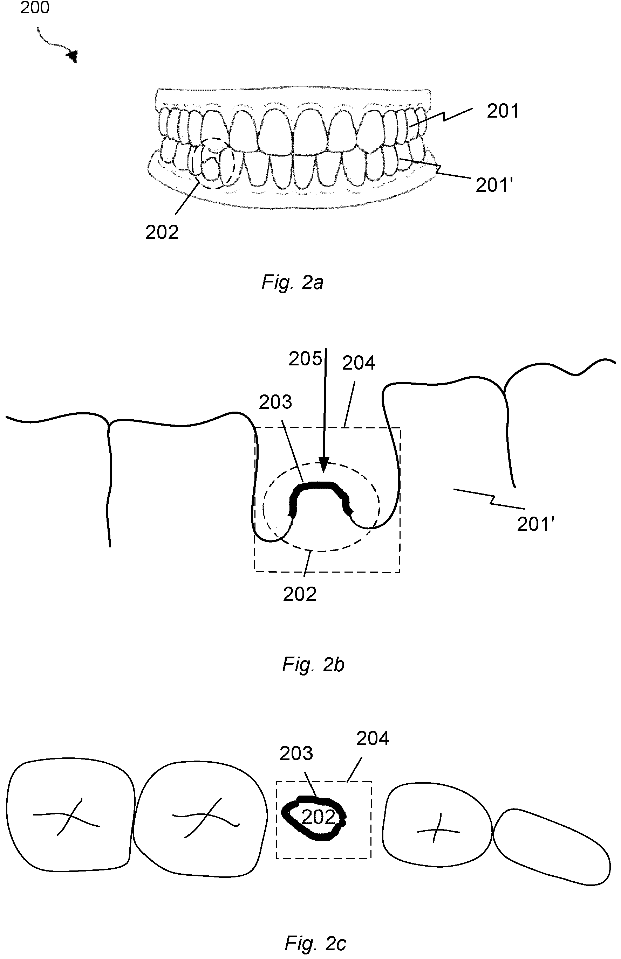

Figures 2a, 2b and 2c illustrate schematic examples of digital 3D surface data 200 of a patient's dentition comprising a preparation surface for use in the computer-implemented method according to the present disclosure. The digital 3D surface data 200 of the patient's dentition may be acquired upon scanning the patient dentition by using an intraoral scanning device. Alternatively, the 3D surface data 200 may be acquired upon obtaining a gypsum impression of the patient's dentition and scanning the gypsum impression using a scanning device. In general, the 3D surface data 200 represents at least a 3D geometry of the patient's dentition. With reference to the example illustrated in Figure 2a, the 3D surface data 200 comprises data describing a surface in 3D space of an upper jaw 201 and data describing a surface in 3D space of a lower jaw 201' of the dentition of the patient. The 3D surface data 200 also comprises information of the upper jaw 201 and of the lower jaw 201' in occlusion, i.e. information of a bite of the patient. Said information may be obtained upon scanning the bite of the patient with the intraoral scanning device, or by scanning a gypsum model of the patient's bite by means of a scanning device. Importantly, accurate bite data enables to design dental restorations that align correctly with the patient's natural bite.

The surface of the dentition comprises a preparation surface 202 which is configured to receive a dental restoration. The dental restoration may be any of one or more of a crown, a veneer, an inlay, and/or an onlay, and so forth. The preparation surface 202 may be obtained upon performing a preparation process on the tooth for which the dental restoration needs to be designed. The preparation process may be performed by a dental practitioner during a clinical visit of the patient, during which the tooth is modified by removing damaged and/or decayed tooth material by means of abrasive rotating dental instruments. In another example (not shown here), the patient's dentition may need multiple dental restorations, e.g. a dental bridge. In this example, the 3D surface data 200 may comprise multiple preparation surfaces, each of them similar to the preparation surface 202 and configured to receive a dental restoration.

Fig. 2b illustrates a side view of the 3D surface data, e.g. as seen from the lingual side or from the buccal side, wherein the preparation surface 202 is visible. Metadata or augmentation data comprising information about the preparation surface and information about the dentition of the patient may be associated with the 3D surface data 200. For example, the metadata or augmentation data may comprise one or more structural features of the preparation surface, such as a location 204 of the preparation surface, and/or a margin line 203 of the preparation surface. The metadata/augmentation data may further comprise one or more design constraints to be enforced when designing the dental restoration, such as an insertion direction 205 of the dental restoration, a planned manufacturing material of the dental restoration, and/or a determined inner surface of the dental restoration. For example, the location 204 of the preparation surface and/or a margin line 203 may be manually determined, i.e. a user may manually mark the location 204 and the margin line 203 on a rendering of the 3D surface data. The manually determined and marked information may be stored as metadata, or the manually marked information may be stored as augmentation data associated with the 3D surface data. In an alternative example, the one or more structural features of the preparation surface and/or the one or more design constraints may be determined based on the obtained 3D surface data 201. For example, the 3D surface data may be processed by using one or more mathematical algorithms or one or more trained models configured to receive at least a part of the 3D surface data as an input and to generate an output comprising the one or more structural features of the preparation surface and/or the one or more design constraints. In a further or alternative example, a combination of metadata/augmentation data and automated algorithms may be used to determine the one or more structural features of the preparation surface and/or the one or more design constraints.

With reference to the example illustrated in Figure 2b, the insertion direction 205 is linear with an angle relative to the normal to the occlusal plane of the jaw 201'. It will be appreciated by those skilled in the art that in some cases the insertion direction may be linear and parallel to said normal, and in other cases the insertion direction may be non-linear, optionally with linear sections.

For illustrative purposes, Figure 2c depicts an occlusal view of the 3D surface data 200, in particular an occlusal view of the lower jaw 201' comprising the preparation surface 202 is illustrated.

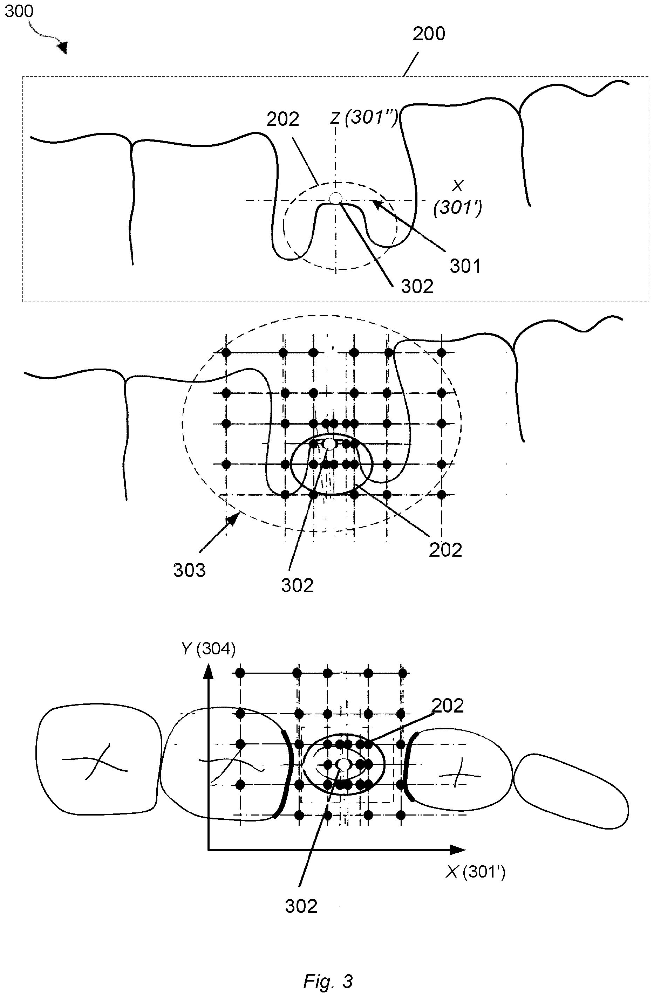

Figure 3 illustrates an example of a schematic workflow 300 to determine a discretization grid according to the present disclosure. Digital 3D surface data 200 of at least a part of a patient's dentition is first obtained, wherein the surface described by the 3D data comprises a preparation surface 202 which is configured to receive the dental restoration. A pose 301 of the preparation surface 302 is either determined based on the digital 3D surface data, or retrieved based on metadata or augmentation data associated with the 3D surface data 200. The pose 301 provides information about the placement and orientation of the preparation surface 302, namely the pose 301 defines a coordinate system for the preparation surface 302. For example, the pose 301 may be determined based on the 3D surface data by. In one example, the pose 301 of the preparation surface may be determined by processing the 3D surface data 200 by means of mathematical algorithms such as Principal Component Analysis (PCA). In another example, the pose 301 may be determined by processing the 3D surface data 200 by means of a trained model, such as a neural network, configured to receive 3D surface data as an input and to generate an output comprising the pose 301 of the preparation surface 202. In yet another example, the pose 301 is retrieved from a metadata file or from a file comprising augmentation data, wherein the file is associated with the 3D surface data 200 and which is stored on a same and/or different storage unit as the 3D surface data 200. For example, the pose 301 of the preparation surface 202 may have been manually or automatically determined and stored on the storage unit as metadata or augmentation data associated with the 3D surface data of the patient.

The pose 301 defines a coordinate system which may be centered in a center 302 of the preparation surface 202, wherein the center 302 may be intended as a centroid of the preparation surface 202. The centroid of the preparation surface may be intended as a point in 3D space that lies in a geometric center of the preparation surface 202. For example, the centroid may be determined by identifying a center of a bounding box placed around the preparation surface. The bounding box may be a 3D box or a cuboid of minimum dimension encompassing the preparation surface. The centroid can be determined by identifying a first point of the bounding box with lowest values of (x, y, z) coordinates a second point of the bounding box with highest values of (x, y, z) coordinates and calculating an average of the first point and second point. The pose of the preparation surface defines a coordinate system comprising an x-axis (301'), a z-axis (301") and a y-axis (not shown in Figure 3). The x-axis (301') may be oriented along a tooth line, wherein the tooth line may be intended as a line tangent to a dental, e.g. the arch of the preparation jaw. The y-axis (not shown in Fig. 3) may be oriented along a lingual or buccal direction, perpendicularly to the x-axis (301'). Therefore, the x-axis 301' and the y-axis strictly depend on the preparation surface 202, and they may generally change with the location of the preparation surface 202. The z-axis 301" is directed towards an occlusal direction of the dentition, and it may be common to all the teeth described by the 3D surface data.

The pose 301 may be used to generate a discretized 3D model of at least a part of the 3D surface data, wherein the at least a part encompasses the preparation surface 202. With reference to the example illustrated in Figure 3, the discretized 3D model comprises a discretization grid 303 centered at the centroid 302 of the preparation surface 202. The discretization grid 303 is structured, such that the points of the grid 303, also referred to as grid points, are arranged following an ordered and predictable pattern. In particular, the grid points may be arranged such that a density of the grid points is larger in a proximity of the center of the grid 302 than at the edge of the grid. Alternatively, the grid points may be equidistant. For example, the density of the grid points may decrease with a distance from the centroid 302. However, it will be appreciated by those skilled in the art that the grid points may be arranged in any other way suitable to define the structured discretization grid 303. Furthermore, even though in the example illustrated in Fig. 3 the discretization grid 303 comprises a plurality of points in 3D space, the discretization grid 303 may comprise any other granularity element suitable to discretize the geometry of a 3D surface. For example, the discretization grid 303 may comprise a plurality of facets, or a plurality of vertices, or a plurality of voxels, and so forth. In another example (not shown here), the discretization grid may be unstructured, for example to model irregular teeth surfaces in an accurate manner.

The discretization grid 303 illustrated in Figure 3 has a finite size, and in particular the size is such that the discretization grid 303 does not encompass the whole 3D surface. In another example, the entire 3D surface data may be discretized depending on the available computational resources and the desired computational resolution of the method disclosed herein. In another example, the size of the discretization grid may be selected based on the pose of the preparation surface 202. In yet another example, the size of the discretization grid may be selected based on a desired resolution of the output of the method according to the present disclosure. In general, the size of the discretization grid 303 may be a predefined feature of the method disclosed herein, or the size of the discretization grid 303 may be manually selected and adjusted by a user and provided as an input to the method.

The lower panel of Fig. 3 illustrates an occlusal view of the discretization grid 303, which illustrates an example of direction of a y-axis 304 of the discretization grid 303. As mentioned above, the y-axis 304 may be directed along the buccal or lingual direction.

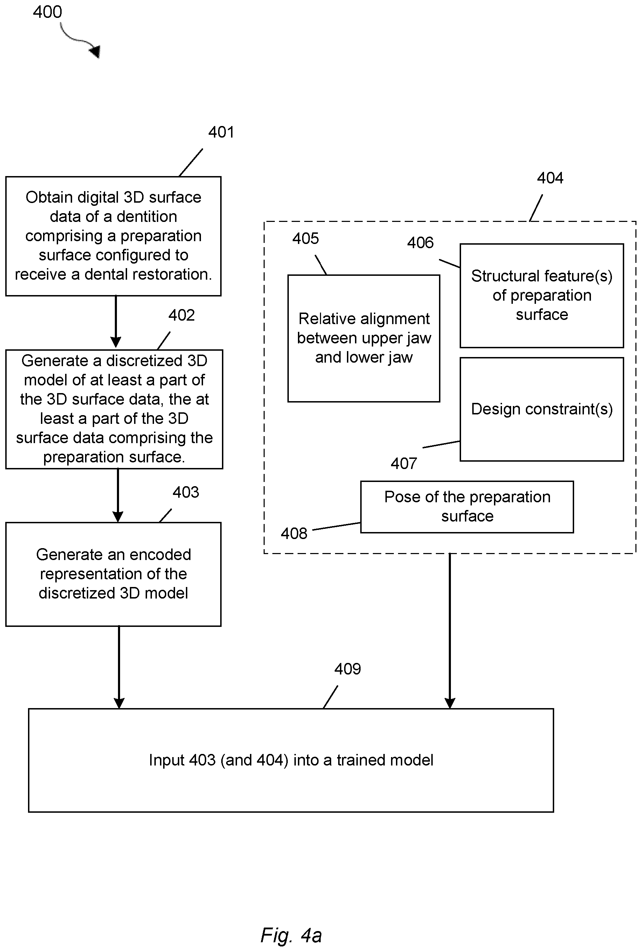

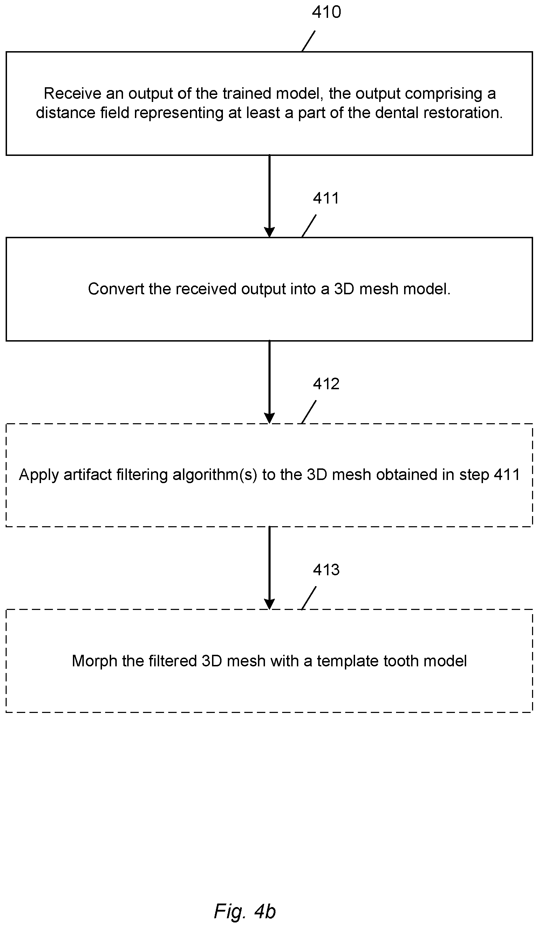

Figures 4a and 4b illustrate a flowchart 400 of a schematic embodiment of the computer-implemented method according to the present disclosure. In step 401, digital 3D surface data of a patient's dentition is obtained, wherein the digital 3D surface data represents a surface of the dentition in 3D space and the surface comprises a preparation surface configured to receive a dental restoration. The digital 3D surface data may comprise a plurality of data points collectively describing at least the geometry in 3D space of the dentition of the patient. The plurality of data points may comprise a point cloud comprising a plurality of points in 3D space, e.g. points described by Cartesian coordinates (x,y,z), or a plurality of facets of a mesh, e.g. triangles in the case of a triangular mesh, or a plurality of vertices of a mesh, or any other data suitable to describe a surface in 3D space.

In step 402, a discretized 3D model of at least a part of the 3D surface data is generated. The at least a part of the 3D surface comprises the preparation surface, such that the discretized 3D model encompasses a discretized representation of the preparation surface. For example, the discretized 3D model of the at least a part of the 3D surface may comprise a discretization grid comprising a plurality of granularity elements, i.e. grid points, collectively describing a discretized representation of the at least a part of the 3D surface. The grid points may be points in 3D space, or facets of a mesh, or vertices of a mesh, or any other finite element suitable to define a discretized representation of at least a part of the surface of the dentition in 3D space.

In step 403, an encoded representation of the discretized 3D model is generated. The encoded representation comprises the grid points, and each grid point is encoded with at least distance information from the point to at least one functional surface. In one example, the at least one functional surface comprises any of one or more of a surface of a preparation jaw, a surface of an antagonist jaw, and/or a minimal surface boundary. In general, the at least one functional surface may be any surface influencing the functionality and/or aesthetic of the patient's dentition, such as its ability to chew, smile, and articulate speech properly. The at least distance information may comprise a distance value, wherein the distance value may be intended as the distance between the grid point and a closest point on the at least one functional surface. In other words, the distance information may be intended as a minimum distance between the grid point and each of the one or more functional surfaces. In one example, the distance value may be determined as an Euclidean distance from the grid point to a closest point on the at least one functional surface, i.e. the distance value may be determined as: $d = min \left[\sqrt{{\left(x-{x}_{i}\right)}^{2} + {\left(y-{y}_{i}\right)}^{2} + {\left(z-{z}_{i}\right)}^{2}}\right]$ wherein (*x, y, z*) are the Cartesian coordinates of the grid point, and (*xᵢ, yᵢ, zᵢ*) are the Cartesian coordinates of the i-th point of the at least one functional surface. In another example, the distance value may be determined as a color distance from the grid point and a closest point to the at least one functional surface, i.e. the distance value may be determined as: $d = min \left[\sqrt{{\left(R-{R}_{i}\right)}^{2} + {\left(G-{G}_{i}\right)}^{2} + {\left(B-{B}_{i}\right)}^{2}}\right]$ wherein (*R, G, B*) are the color coordinates of the grid point in red, green, blue (RGB) space, and (*Rᵢ, Gᵢ, Bᵢ*) are the color coordinates of the i-th point of the at least one functional surface in RGB space. In general, the distance information is not limited to the above-mentioned examples, and the distance information may comprise any other distance definition suitable to measure a distance between each grid point and the at least one functional surface and to generate an encoded representation of the discretized 3D model based on the distance, e.g. the distance may be measured in a polar coordinates system. In some examples, the encoded representation of the discretized 3D model generated in step 403 may be a distance field representation. In another example, the encoded representation may be a binary representation in which each grid point is encoded with a sign "+" or a sign "-", depending on whether the grid point is located outside or inside the at least one functional surface, respectively.

In step 409, the encoded representation of the discretized 3D model generated in step 403 is input into a trained model. The trained model may receive as an input in step 409 further data 404, wherein the data 404 may comprise one or more of a relative alignment between upper jaw and lower jaw 405, one or more structural features of the preparation surface 406, one or more design constraints to be enforced when designing the dental restoration, and/or a pose of the preparation surface 408. The relative alignment between the upper jaw and the lower jaw 405 may be obtained during a scanning session of the dentition of the patient, e.g. the scanning session during which the 3D surface data of the dentition is obtained by means of an intraoral scanner. For example, the relative alignment between the upper jaw and the lower jaw may be obtained upon scanning the upper jaw and lower jaw in occlusion, i.e. by scanning a bite of the patient. Alternatively, the relative alignment between the upper jaw and the lower jaw may be obtained upon scanning a gypsum model of the upper jaw and lower jaw in occlusion by means of a scanning device. The one or more structural features of the preparation surface 406 may comprise one or more of a location of the preparation surface and/or a margin line of the preparation surface. The one or more design constraints 407 to be enforced when designing the dental restoration may comprise one or more of an insertion direction of the dental restoration, a planned manufacturing material of the dental restoration, and/or a determined inner surface of the dental restoration. For example, the inner surface of the dental restoration may be determined based on the planned manufacturing material of the dental restoration and/or based on a layer of cement or glue which intended for application on the preparation surface to mount the dental restoration in place. The pose of the preparation surface 408 may be intended as a local coordinate system for the preparation surface, where the local coordinate system comprises an origin located in a center of the preparation surface and three mutually orthogonal axes intersecting at the origin. The center of the preparation surface may be intended as a geometric center, i.e. as a centroid of the preparation surface. The pose of the preparation surface 408 may be used to generate the discretization of the at least a part of the 3D surface data.

**In** one example, the data 404 is metadata or augmentation data associated with the digital 3D surface data obtained in step 401, i.e. the data 404 is stored in a metadata file or augmentation data file associated with the digital 3D surface data. For example, one or more of the relative alignment between the upper and lower jaw 405, structural feature(s) and/or design constraint(s) may be manually determined by a dental practitioner. For example, the dental practitioner may manually determine the location of the preparation surface and mark it on a graphical rendering of the 3D surface data. Accordingly, the marked location may be stored as metadata associated with the 3D surface data of the patient. Furthermore, the dental practitioner may select the planned manufacturing material of the dental restoration and provide it as metadata or augmentation data associated with the digital 3D surface data to the trained model in 408. In general, at least a part of the data 404 may be manually determined by a dental practitioner, stored in a storage unit as metadata file(s) or augmentation data file(s) associated with the patient's specific 3D surface data and obtained upon downloading said metadata file(s) from the storage unit. In yet another example, at least a part of the data 404 may be automatically determined based on the obtained 3D surface data, by processing the 3D surface data using one or more algorithms configured to determine the one or more structural features 406, the one or more design constraints 407, and/or the pose of the preparation surface 408. For example, the pose of the preparation surface 408 may be automatically determined by using mathematical methods such as Principal Component Analysis (PCA), or by using a trained model such as a neural network trained to process the 3D surface data and to determine the pose of a tooth and preparation surface. In yet another example, a part of the data 404 may be manually determined and stored on metadata or augmentation data file(s), e.g. comprising information which is manually determined and/or annotated by a dental practitioner, and partially determined by processing the obtained 3D surface data, e.g. using mathematical methods and/or specific trained models. Independently on whether the data 404 is obtained or retrieved, one or more of the data 404 may be input to the trained model in step 409. The trained model may be a trained neural network model, such as a Deep Convolutional Neural Network. Further details about the trained model used in step 409 will be provided later in the detailed description of the present application.

Figure 4b illustrates a schematic example of a continuation of the workflow illustrated in Figure 4a. In step 410, an output of the trained model used in step 409 is received, wherein the output may comprise at least a part of the dental restoration. For example, the output may comprise an outer surface of the dental restoration. In another example, the output of step 409 may comprise both an outer surface of the dental restoration and an inner part of the dental restoration. The output of the trained model may comprise an encoded representation of the at least a part of the dental restoration. For example, the encoded representation of the at least a part of the dental restoration may be a distance field representation of the at least a part of the dental restoration. In another example, the encoded representation of the at least a part of the dental restoration may be a binary representation of the at least a part of the dental restoration. In general, the output of the trained model may comprise an encoded representation similar to the encoded representation input in the trained model in step 409. With reference to the example illustrated in Figure 4b, the output of the encoded representation is a distance field representing at least a part of the dental restoration, such as an outer surface of the dental restoration.

In step 411, the output of the trained model may be converted to a mesh model. For example, the output of the trained model may be a distance field representation of the outer surface of the dental restoration, which may be converted, in step 411, to a 3D triangular mesh representation. The conversion from the encoded representation to the mesh model representation may comprise processing the output of the trained model using a distance field to surface mesh algorithm, such as a marching cubes algorithm.

In step 412, an artifact filtering algorithm(s) is applied to the3D mesh obtained in step 411. For example, the artifact filtering algorithm(s) may comprise smoothing one or more surface irregularities comprised in the output of the trained model. For example, the artifact filtering algorithm(s) may comprise a Laplacian smoothing algorithm. Step 412 may be optionally performed depending on the resolution of the output of the trained model and depending on the desired resolution of said output.

In step 413, the output of the trained model is morphed with a template tooth model to refine one or more anatomical features of the dental restoration, e.g. to refine the ridges and the grooves of the dental restoration. The tooth template model may be a digital template of a specific tooth type for which the dental restoration needs to be designed, e.g. a template of a digital molar if a molar of the patient's dentition requires the dental restoration. The template tooth model may be stored in a digital dental library comprising a digital model of each tooth type in the dentition, and the specific tooth type may be retrieved from the digital dental library based on the tooth for which the dental restoration is designed. In one example, morphing the output of the trained model with the template tooth may comprise using a non-linear Iterative Closest Point Method (non-linear ICP) to align the 3D outer surface of the dental restoration predicted by the trained model with the template tooth model. For example, the non-linear ICP algorithm may be built upon a Conjugate Gradient optimizer based on measuring distances between the output of the trained model and template tooth in a closest point to plane method, where the planes are determined by the surface normal, in a point to point method, and/or in a point to surface method. Similarly to step 412, step 413 may be optionally performed depending on the resolution of the received output of the trained model and based on the desired resolution of the at least a part of the dental restoration. Step 411-413 of Figure 4b may be performed to digitally design the dental restoration.

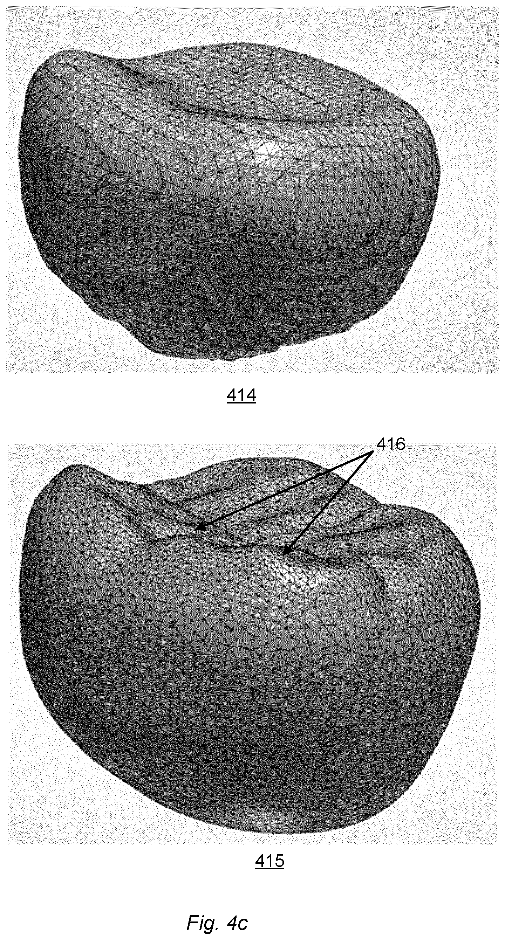

Figure 4c represents an example of an output 414 of the trained model according to the present disclosure, upon converting the output to a mesh model. The mesh model 414 may have been optionally processed by using one or more artifact filtering algorithms according to the discussion above. After conversion to a mesh model and after artifact filtering, the output 414 may be morphed with a digital tooth template model to digitally design the dental restoration 415. The digitally designed dental restoration 415 comprises more refined anatomical features 416, e.g. ridges and grooves, when compared to the mesh model 414. However, in some examples the digitally designed dental restoration 415 may be obtained upon converting the output of the trained model into a mesh model, i.e. without the need of performing the morphing step 413. This may depend on one or more properties of the trained model, such as the resolution of the trained model.

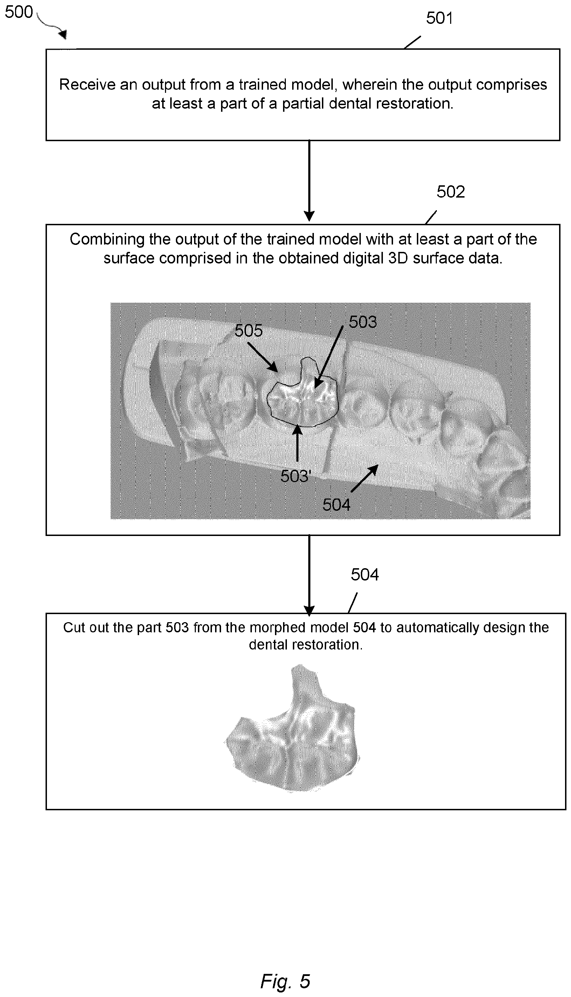

Figure 5 illustrates a schematic example of a part 500 of a workflow for designing a partial dental restoration according to the present disclosure. In step 501, an output is received from a trained model, wherein the output comprises at least a part of a partial dental restoration. For example, the trained model may be a trained neural network which is configured to receive as an input 3D surface data of a patient's dentition comprising a preparation surface which is configured to receive the partial dental restoration, and to output a prediction of a shape of at least a part of the partial dental restoration. In another example, the trained model may be a trained neural network which is configured to receive as an input an encoded representation of a discretized 3D model, wherein the discretized 3D model is a discretized representation of at least a part of the 3D surface data describing the patient's dentition. The trained neural network may be configured to output an encoded representation of at least a part of the partial dental restoration. For example, the output received in step 501 may comprise an encoded representation of an outer surface of the dental restoration, and/or the output received in step 501 may comprise an encoded representation of an inner structure of the dental restoration. The output of the trained model received in step 501 may be obtained according to the computer-implemented method of the present disclosure, e.g. according to the method illustrated as reference number 100 in Figure 1. The partial dental restoration may be intended as any of one or more of a veneer, an inlay, an onlay, and so forth. In general, the partial dental restoration may be any dental restoration which aims at restoring only a part of a damaged tooth, e.g. restoring cavities in the tooth or restoring a damaged surface of the tooth.

In step 502, the output of the trained model received in step 501 is merged with at least a part of the surface comprised in the obtained 3D surface data to automatically design the partial dental restoration. For example, the at least a part of the surface comprised in the obtained 3D surface data may comprise a part of a tooth which is intact after a preparation process has been performed on the tooth, wherein the tooth is the tooth configured to receive the dental restoration. In another example, 3D surface data relative to a tooth acquired during a scanning session before the tooth suffered from any damage may exist and be stored in a storage unit, wherein the tooth is the tooth configured to receive the dental restoration. Accordingly, the at least a part of the surface may comprise the 3D surface data of the tooth before the tooth suffered of any damage. In yet another example, the partial dental restoration is a veneer, and only the buccal surface of the tooth configured to receive the veneer is removed, whereas the lingual part is intact. Therefore, the output of the trained model may be merged with the lingual part of the tooth in the 3D surface data. In yet another example, the partial dental restoration is an inlay, and only a portion of the tooth is prepared to receive the dental restoration, while one or more of the buccal surface, lingual surface, and/or interproximal surface are intact. Therefore, in step 502 the output of the trained model received in step 501 may be merged with the one or more intact surfaces described by the 3D surface data.

An exemplary visual representation of step 502 is illustrated in Figure 5. The output of the trained model is merged with a part of the 3D surface data comprising the surface of the tooth 505 which is intact after the preparation process, wherein the tooth 505 is the tooth in need of the dental restoration. Accordingly, a morphed model 504 of the 3D surface data with the output of the trained model is obtained. The output portion 503 represents the missing part of tooth 505 in the 3D surface data of tooth 505, therefore the output portion 503 corresponds to the dental restoration that needs to be designed. In other words, the 3D surface data relative to the tooth 505 lack information about the surface portion 503 which is instead encompassed in the output of the trained model. The output of the trained model received in step 501 may comprise a prediction of an outer surface of the entire tooth 505. In yet another example, the output of the trained model received in step 501 may coincide with the missing part of the tooth surface 505, i.e. the output portion 503 may coincide with the output of the trained model. In this case, the morphing of the output with at least a part of the surface data performed in step 502 may be performed to refine the details of the predicted dental restoration, e.g. to refine the margin line 503' to ensure that the restoration properly fits the tooth for which the restoration is designed. Accordingly, in step 506 the output portion 503 is cut out from the morphed model 505 to digitally design the dental restoration. In some examples, further post-processing may be performed on the digitally designed dental restoration obtained in step 506, e.g. to refine one or more anatomical features, such as grooves and ridges, of the dental restoration.

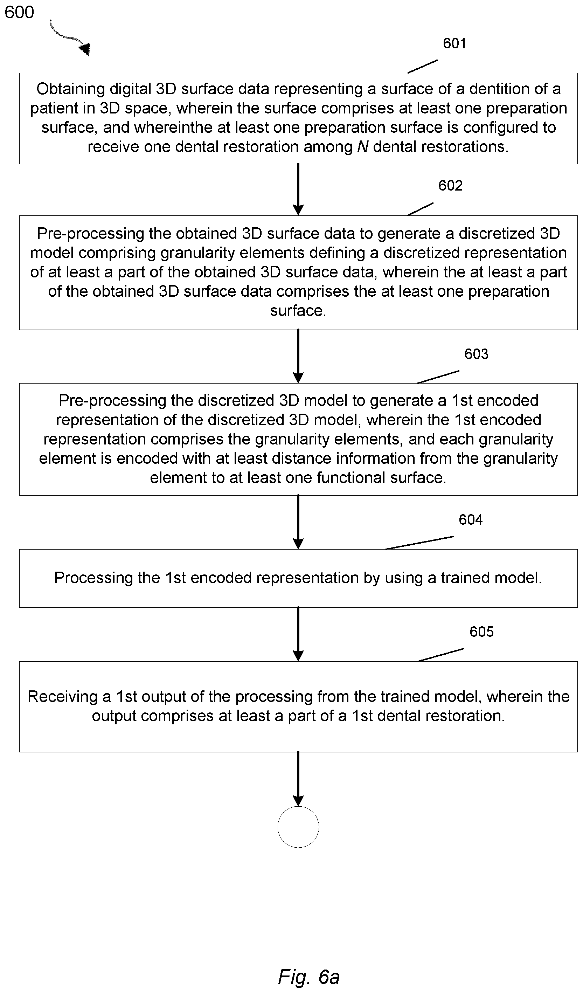

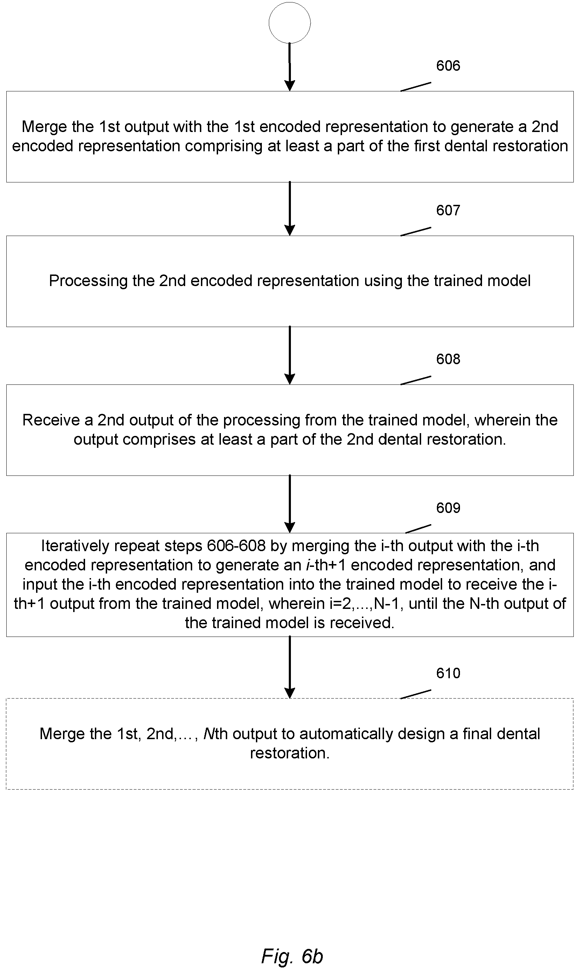

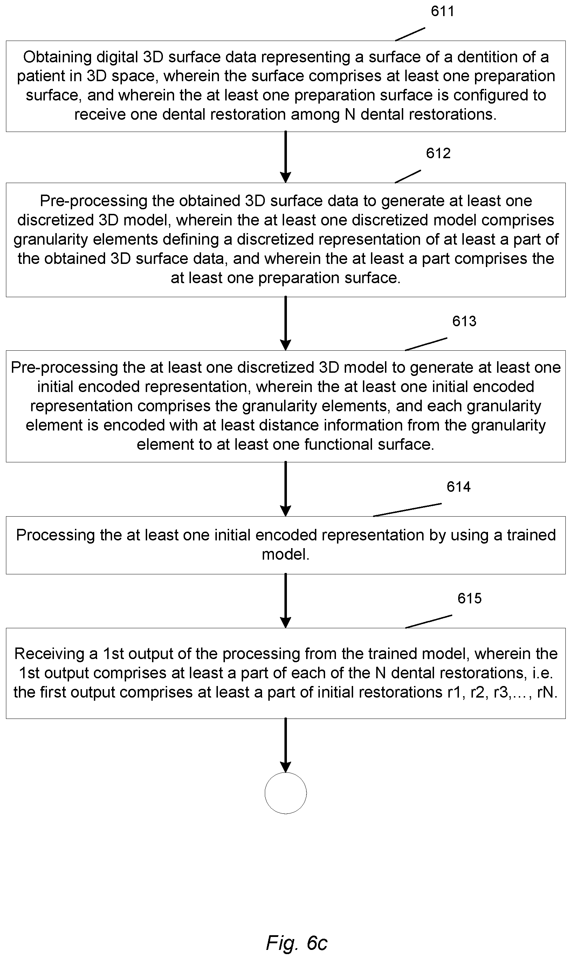

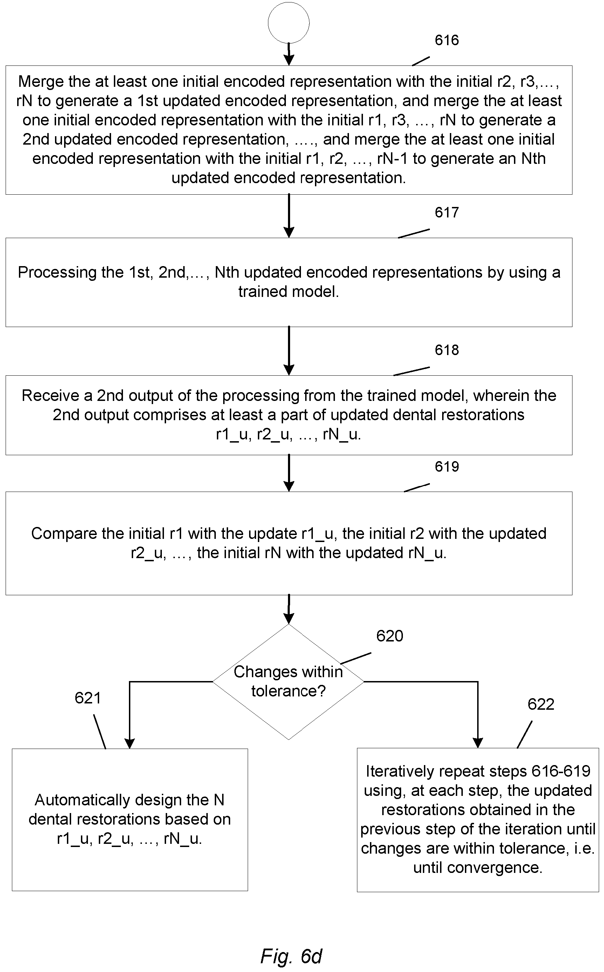

Figures 6a, 6b, 6c and 6d illustrate a schematic flowchart 600 of an embodiment of a workflow to design multiple dental restorations according to the present disclosure. In step 601, digital 3D surface data representing a surface of a dentition patient in 3D space is obtained. The surface described by the 3D surface data comprises at least one preparation surface, and the at least one preparation surface is configured to receive one dental restoration among N dental restorations. For example, multiple teeth in the patient's dentition may be damaged, either completely or partially, and therefore the patient may necessitate multiple crowns, veneers, inlays, onlays, and so forth. In this case, the surface described by the 3D surface may comprise N preparation surfaces, and each preparation surface may be configured to receive a dental restoration among the N dental restorations. In another example, 3 dental restorations may be connected to form a dental bridge, where the middle tooth is completely missing, and the two outside teeth are prepared to receive a crown each, i.e. the 3D surface data may comprise two preparation surfaces.

In step 602, the obtained 3D surface is pre-processed to generate a discretized 3D model comprising granularity elements defining a discretized representation of at least a part of the obtained 3D surface data, wherein the at least a part of the obtained 3D surface data comprises the at least one preparation surface. For example, the discretized 3D model may be a discretization grid encompassing the at least one preparation surface. In particular, the discretization grid may be a structured discretization grid centered at a central location of the preparation surface, wherein the central location may be understood as a centroid of the preparation surface. The location of the at least one preparation surface within the discretization grid may be determined based on a pose of the preparation surface, wherein the pose may be either determined based on the 3D surface data or it may be retrieved, e.g. the pose of the 3D surface data may be manually determined by a dental practitioner and the manually determined pose may be stored as metadata or augmentation data associated with the 3D surface data. The granularity elements may be referred to herein as grid points. The grid points may be the points of a 3D point cloud, i.e. each grid point may be identified by its Cartesian coordinate (x, y, z). In other examples, the grid points may be facets of a polygonal mesh, or vertices of a polygonal mesh, or voxels in 3D space, or any other finite element which is suitable to define a discretized representation of a surface in 3D space.

In step 603, the discretized 3D model is pre-processed to generate a 1^{st} encoded representation of the discretized 3D model. The 1^{st} encoded representation comprises the grid points, and each grid point is encoded with at least distance information from the point to at least one functional surface. For example, the distance information may comprise a distance value from each grid point to the at least one functional surface, i.e. the 1^{st} encoded representation may be a distance field representation of the discretized 3D model. Therefore, each grid point may be encoded with a distance value, wherein the distance value may be positive if the grid point is located outside the at least one functional surface, or the distance value may be negative if the grid point is located inside the at least one functional surface, or the distance value may be zero if the grid point is located on the at least one functional surface. The distance value may be determined by calculating the Euclidean distance of each grid point to the corresponding closest point, in the Euclidean space, on the at least one functional surface. In another example, the distance value may be determined by calculating the color distance of each grid point to the corresponding closest point, in the color space, on the at least one functional surface. In another example, the encoded representation may be a truncated distance field, similar to the distance field disclosed above but comprising only grid points for which the distance value from the corresponding point to the at least one functional surface is smaller than a predefined distance threshold. In other words, the truncated distance field comprises only the grid points which are located at a distance from the at least one functional surface which is within a predefined range of distance values, wherein the predefined range may be defined as a truncation range and may be based on a desired resolution of the method. In another example, the encoded representation may be a binary representation, in which each grid point is encoded with a "+" or "-" sign, depending on whether the point is located outside or inside the at least one functional surface. In general, the 1^{st} encoded representation may be any representation suitable to encode at least distance information of each grid point to the at least one functional surface. The at least one functional surface comprises one or more of a surface of a preparation jaw, a surface of an antagonist jaw, and/or a minimal boundary surface.

In step 604, the 1^{st} encoded representation is processed by using a trained model. The trained model may comprise a neural network which is configured to process encoded representations similar to the 1^{st} encoded representation. For example, the trained neural network may be configured to process distance field representations. In another example, the trained model may be a neural network configured to process binary representations. In general, the trained model may be a trained neural network, such as a Deep Convolutional Neural Network, which is configured to receive as an input an encoded representation similar to the 1^{st} encoded representation and to generate an output comprising at least a part of a dental restoration. The output may be in the form of an encoded representation having a similar structure to the input encoded representation.

In step 605, a 1^{st} output of the processing from the trained model is received, wherein the output comprises at least a part of a 1^{st} dental restoration. For example, the output may comprise a prediction of an outer surface of the 1^{st} dental restoration. The output may further or alternatively comprise a prediction of the outer surface and a prediction of the inner structure of the first dental restoration. As mentioned above, the output may be in the form of an encoded representation having a similar structure to the input encoded representation. For example, if the 1^{st} encoded representation input in the trained model in step 604 is a distance field representation, the 1^{st} output received in step 605 may be a distance field representation of the at least a part of the 1^{st} dental restoration. Furthermore, the resolution of the output of the trained model received in step 605 may be the same as the resolution of the input of the trained model. Therefore, the resolution of the output may be controlled upon changing the resolution of the input to the trained model, which in turn depends on the resolution of the discretized model generated in step 602. In other words, the resolution of the discretized model generated in step 602 may be selected based on a desired resolution of the output of the trained model. Alternatively, the resolution of the discretized model generated in step 602 may be pre-selected based on a desired and fixed resolution of the output of the trained model and set as a predefined feature of the method described herein.

Figure 6b illustrates a continuation of the workflow illustrated in Figure 6a, which may be performed after step 605 of Figure 6a. In step 606, the 1^{st} output of the trained model obtained in step 605 is merged with the 1^{st} encoded representation to generate a 2^{nd} encoded representation which comprises at least a part of the first dental restoration. The 1^{st} encoded representation is the same encoded representation which is input in the trained model in step 604, namely the encoded representation which is fed into the trained model to generate the at least a part of the 1^{st} dental restoration. Furthermore, the 2^{nd} encoded representation has the same structure as the 1^{st} encoded representation. For example, if the 1^{st} encoded representation is a distance field representation, then the 2^{nd} encoded representation is a distance field representation comprising a distance field representation of the at least a part of the first dental restoration. It will appear clear to those skilled in the art that distance field representations are particularly advantageous from a computational point of view in the merging step 606, as the underlying mathematical operations of the merging step 606 comprise Boolean operations, e.g. union of multiple geometrical shapes, which can be easily implemented when dealing with distance fields.

At this point, the workflow is iteratively repeated. In step 607, the 2^{nd} encoded representation is fed into the trained model. The trained model used in step 607 is the same as the trained model used in step 604 to process the 1^{st} encoded representation. Therefore, the above-mentioned discussion and considerations about the trained model also hold for step 607.

In step 608, a 2^{nd} output of the processing from the trained model is received. The 2^{nd} output of the processing comprises at least a part of the 2^{nd} dental restoration. For example, the output 608 may comprise an outer surface of the 2^{nd} dental restoration, or the output 608 may comprise both an outer surface and an inner structure of the 2^{nd} dental restoration. Similarly to the 1^{st} output obtained in step 605, the 2^{nd} output may be in the form of an encoded representation similar to the 2^{nd} encoded representation input in the trained model in step 607. For example, the 2^{nd} output may be a distance field comprising a distance field representation of the at least a part of the second dental restoration.

In step 609, steps 606-608 are iteratively repeated until at least a part of the *N*-th dental restoration is received as an output from the trained model. In particular, step 609 comprises the steps of:
a. merging the *i*-th output of the trained model with the *i*-th encoded representation;
b. input the *i*-th encoded representation into the trained model to generate an *i-*th+1encoded representation;
c. input the *i*-th+1 encoded representation into the trained model; and
d. receive an *i*-th+1 output from the trained model;
e. repeat steps a,..,d for a number of *i*=2,...,*N-*1 times until the *N-*th output of the trained model is received.

In other words, the output from the trained model received at each step of the method is merged with the existing encoded representation and input in the trained model to generate the next dental restoration. As mentioned above, the advantages of using distance fields as encoded representations will be appreciated by those skilled in the art, since distance fields are particularly suitable for Boolean computations such as the union of multiple geometrical shapes.

In step 610, the 1^{st}, 2^{nd}, 3^{rd},..., *N*-th outputs may be merged to design the final dental restoration. Step 610 may be performed, for example, when the final dental restoration is a dental bridge, e.g. a bridge comprising one or more crowns and/or one or more implants. In some cases, multiple teeth of the patient's dentition may be damaged, and said damaged teeth may be not connected or adjacent to each other. For example, the patient may need a crown for a molar and a veneer for an incisor. In this case, the method illustrated in Figures 6a-6b may be performed to design multiple dental restorations which are not connected with each other, i.e. steps 601-609 may be performed to design said dental restorations. After performing the iterative method, i.e. after receiving the N-th output from the trained model, each of the 1^{st}, 2^{nd},..., *N*-th dental restorations may be post-processed to refine the anatomical features of each dental restoration. For example, the post-processing may comprise converting each of the 1^{st}, 2^{nd},..., *N*-th outputs into a mesh model and morphing each of the 1^{st}, 2^{nd},..., *N*-th converted outputs with a digital tooth template, e.g. to refine one or more anatomical features of the dental restoration such as the ridges and grooves of the dental restoration. If step 610 is performed to merge the 1^{st}, 2^{nd},..., *N*-th dental restorations to form a dental bridge, then the resulting dental bridge may be converted to a mesh model and optionally morphed with a digital dental bridge template to refine the anatomical features of the dental bridge. For example, the digital tooth template and/or the digital dental bridge template may be stored in a digital dental repository which may comprise one or more templates for each tooth in the patient's dentition. The digital template(s) may be therefore selected based on the tooth or teeth for which the dental restoration is designed.

Figures 6c and 6d illustrate an embodiment of a workflow to design multiple dental restorations, alternative or further to the workflow illustrated in Figures 6a and 6b, according to the present disclosure. In step 611, digital 3D surface data representing a surface of a dentition of a patient in 3D space is obtained. The 3D surface comprises at least one preparation surface which is configured to receive one dental restoration among *N* dental restorations to be designed. In general, when *N* dental restorations need to be designed for the patient dentition, the 3D surface data may comprise *N* dental restorative sites, wherein each restorative site is configured to receive one among the *N* dental restorations, and wherein the *N* restorative site may comprise the at least one preparation surface. For example, the patient may have a missing tooth and may need a dental bridge. In one example, the 3D surface may comprise two preparation surfaces and a restorative site, wherein the restorative site is located between the two preparation surfaces and is configured to receive a full restorative tooth, whereas each of the two preparation surfaces may be configured to receive a dental crown. The two dental crowns may be connected through the restorative tooth to form the dental bridge. In another example, the 3D surface may comprise a combination of at least 2 preparation surfaces and 0 or more restorative sites, wherein the restorative sites are configured to receive a full restorative tooth and the restorative surfaces are configured to receive restorative crowns. The restorative crowns and restorative teeth may be connected to form the dental bridge. In other cases, the patient may have multiple damaged teeth which are not neighboring teeth. Accordingly, the 3D surface may comprise multiple preparation surfaces.

In step 612, the obtained 3D surface data is pre-processed to generate at least one discretized model comprising granularity elements defining a discretized representation of at least a part of the obtained 3D surface data. The at least a part of the 3D surface data comprises the at least one preparation surface. With reference to one of the examples of the dental bridge described above, a discretized representation comprising the two restorative sites and the preparation surface may be generated in step 612. In another example, three different discretized representations may be generated in step 612, namely a discretized representation comprising one of the two restorative sites, a discretized representation comprising the preparation surface, and a discretized representation comprising the other restorative site. In practice, either one discretized 3D model comprising all the restorative sites and the at least one preparation surface may be generated in step 612, or multiple discretized 3D models may be generated in step 612, one for each of the *N* dental restorations to design. Each of the discretized 3D model may be centered at a location of the corresponding restorative site or of the corresponding preparation surface, namely each discretized 3D model may be a structured discretization grid centered at a centroid of the corresponding restorative site or preparation surface.

In step 613, the at least one discretized 3D model is pre-processed to generate at least one initial encoded representation, wherein the at least one encoded initial representation comprises the granularity elements and each granularity element is encoded with at least distance information from the granularity element to at least one functional surface. Therefore, if a single discretized 3D model is generated in step 612, then a single encoded representation is generated in step 613. Otherwise, an encoded representation for each of the discretized 3D models is generated in step 613, i.e. an encoded representation for each of the *N* dental restorations to design. Possible embodiments of the at least distance information and of the at least one functional surface are the same as the one described above in the detailed description, e.g. in the detailed description of Figure 6a and 6b. The at least one encoded representation may be a distance field representation of the at least one discretized 3D model, wherein each granularity element is encoded with a distance value from the granularity element to a closest point on the at least one functional surface. In another example, the at least one encoded representation may be a binary representation wherein each granularity element is encoded with a sign "+" or a sign "-" depending on whether the granularity element is located outside or inside the at least one functional surface, respectively.

In step 614, the at least one initial encoded representation is processed by using a trained model. For example, the trained model may be a Deep Convolutional Neural Network configured to process encoded representations similar to the at least one initial encoded representation. For example, if the at least one initial encoded representation is a distance field representation, the Deep Convolutional Neural Network is configured to process distance field representations.

In step 615, a 1^{st} output of the processing is received from the trained model, wherein the 1^{st} output comprises at least a part of each of the *N* dental restorations, i.e. the first output comprises at least a part of each of the initial restorations *r*₁*, r*₂*, r*₃*, ..., r_{N}.* In other words, the output received in step 615 may comprise an initial prediction of an outer shape and/or an initial prediction of an inner structure of each of the *N* dental restorations to design. Said initial prediction may be in the same form as the input of the trained model, e.g. the initial prediction may be an encoded representation with a same structure as the at least one initial encoded representation input in the trained model in step 614.

Figure 6d illustrates a continuation of the workflow illustrated in Figure 6c. In step 616, the at least one initial encoded representation is merged with the initial *r*₂, *r*₃*, ..., r_{N}* to generate a 1^{st} updated encoded representation. The 1^{st} updated encoded representation comprises the initial predictions for the restorations *r*₂*, r*₃*, ..., r_{N}.* Similarly, in step 616 the at least one initial encoded representation is merged with the initial *r*₁*, r*₃*, ..., r_{N}* to generate a 2^{nd} updated encoded representation comprising the initial predictions for the restorations *r*₁*, r*₃*, ..., r_{N}.* In step 616, the merging process is performed until the *N^{th}* updated encoded representation is generated upon merging the at least one initial encoded representation with the initial *r*₁*, r*₂, *..., r*_{*N-*1}*.* If an initial encoded representation is generated for each of the N dental restorations in step 613 of Figure 6c, then step 616 may comprise merging the initial encoded representation input in the trained model to generate a given dental restoration with the remaining *N* - 1 initial restoration. For example, if a first initial encoded representation is input in the trained model in step 614 to generate the initial dental restoration *r*₁, in step 616 the first initial encoded representation may be merged with the initial *r₂, r₃, ..., r_{N}* to generate a first updated encoded representation. Similarly, if a second initial encoded representation is input in the trained model in step 614 to generate the initial dental restoration *r*₂, in step 616 the second initial encoded representation may be merged with the initial *r*₁*, r*₃*, ..., r_{N}* to generate a second encoded representation. The merging process may be repeated up to the *N^{th}* initial encoded representation, which may have been input in step 614 to generate the initial *r_{N}.* Accordingly, the *N^{th}* initial representation may be merged with the initial *r*₁*, r*₂*, ..., r*_{*N*-1} to generate an *N^{th}* updated encoded representation. In general, in step 616 a set of updated encoded representations may be obtained, wherein the set comprises the updated representations obtained for each of the *N* initial encoded representations.

In step 617, the 1^{st}, 2^{nd} *..., N^{th}* updated encoded representations are processed by using a trained model. The trained model may be the same as the trained model used in step 614 of the method. In step 618, a 2^{nd} output of the processing is received from the trained model, wherein the 2^{nd} output comprises at least a part of each of the updated dental restorations *r*₁*ᵤ, r*₂*ᵤ, r*₃*ᵤ, ... , r_{Nu}.*

In step 619, the initial dental restorations obtained in step 615 are compared with the updated dental restorations obtained in step 618, namely the initial *r*₁ is compared with *r*₁*ᵤ,* the initial *r*₂ is compared with the updated *r*₂*ᵤ,* and so forth, up to the initial *r_{N}* which is compared with the updated *r_{Nu}.* For example, the comparison may comprise determining a difference between the encoded information in each granularity element of a given initial restoration with the encoded information in a corresponding granularity element of the corresponding updated restoration. In step 620, it is determined whether the changes, i.e. differences, between the initial dental restorations and the updated dental restorations are within a predefined tolerance. For example, the predefined tolerance may be a numerical threshold which is a set feature of the method. In another example, the predefined tolerance may be a numerical threshold which may be adapted in the method based on a desired convergence criterion. The convergence criterion may be based, for example, on a desired accuracy of the method of the present disclosure.

If in step 620 it is determined that the convergence criterion is satisfied, then in step 621 the *N* dental restorations may be automatically designed based on the updated *r*₁*ᵤ, r*₂*ᵤ, ..., r_{Nu}.* For example, step 621 may comprise converting the updated *r*₁*ᵤ, r*₂*ᵤ,* ..., *r_{Nu}* to a mesh model, if the updated restorations are obtained in the form of encoded representations. Automatically designing the *N* dental restorations may further comprise morphing each of the updated restorations *r*₁*ᵤ, r*₂*ᵤ,* ..., *r_{Nu}* with a digital tooth template to refine one or more anatomical features of each of the dental restorations. Automatically designing the *N* dental restorations may further or alternatively comprise merging the updated *r*₁*ᵤ, r*₂*ᵤ, ..., r_{Nu}* into a single dental restoration, e.g. to form a dental bridge.

If in step 621 it is determined that the convergence criterion is not satisfied, then steps 616-619 of the method are iteratively repeated using, at each step, the updated restorations obtained in the previous step of the iteration, until the changes between two consecutive steps are within the tolerance, i.e. until convergence of the method. At this point, step 621 may be performed as described above with the updated dental restorations obtained in the last iteration of the method.

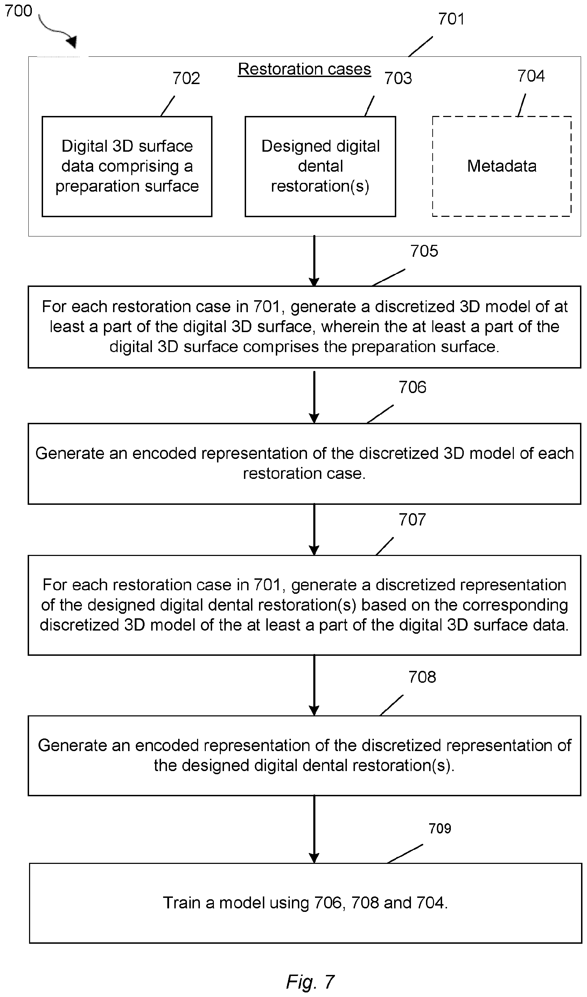

Figure 7 illustrates a flowchart 700 of a schematic example of a method for training a model according to the present disclosure. The training method of the model according to the present disclosure is based on a plurality of restoration cases 701, wherein each restoration case comprises: i) digital 3D surface data of a patient's dentition 702, the surface comprising a preparation surface configured to receive a dental restoration(s); ii) the designed digital dental restoration(s) 703 corresponding to the digital 3D surface data 702; and iii) metadata 704 associated with the digital 3D surface data 702. For each 3D surface data 702, the corresponding designed digital dental restoration(s) 703 may have been manually designed by a dental practitioner, or the designed digital dental restoration(s) 703 may have been automatically designed by using a software configured to design dental restorations, and subsequently the automatically designed digital dental restoration(s) may have been manually refined by a dental practitioner to obtain the designed digital dental restoration(s) 703. The metadata 704 may comprise information about one or more properties/features of the digital 3D surface data, and/or information about one or more properties/features about the preparation surface, and/or information about one or more properties/features about the designed digital dental restoration(s). For example, for a given restoration case among the restoration cases 701, the metadata 704 may comprise a relative alignment between an upper jaw and a lower jaw described by the digital 3D surface data. The metadata 704 may further or alternatively comprise one or more of a location of the preparation surface in the corresponding digital 3D surface data 702, a margin line of the preparation surface comprised in the corresponding digital 3D surface data 702, an insertion direction of the corresponding designed dental restoration 703, a planned manufacturing material of the designed dental restoration 703, and/or a determined inner surface of the dental restoration 703. In general, the metadata 704 may comprise information about any structural features of the corresponding preparation surface and/or any design constraints which have been enforced in the design process of the designed dental restoration(s) 703.

The following steps may be performed for each restoration case in the plurality of restoration cases 701. In step 705, for each restoration case in 701, a discretized 3D model of at least a part of the digital 3D surface data 702 is generated, wherein the at least a part of the digital 3D surface comprises the preparation surface. The discretized 3D model of at least a part of the digital 3D surface data 702 may comprise a structured discretization grid, the grid comprising a plurality of granularity elements/grid points. The discretization grid may be centered at a location of a centroid or a center of mass of the corresponding preparation surface. In general, a position of the preparation surface within the discretization grid may be based on a pose of the preparation surface. For each restoration case in 701, the pose of the preparation surface may be determined based on the corresponding 3D surface data 702, or the pose of the preparation surface may be retrieved from the corresponding metadata 704. For example, the pose of the preparation surface of each restoration case in 701 may have been manually estimated by a dental practitioner, e.g. by marking the pose on a graphical rendering of the corresponding 3D surface data 702, and stored as metadata 704.

In step 706, an encoded representation of the discretized 3D model of each restoration case is generated. The encoded representation of each discretized 3D model comprises the grid points of the corresponding discretized 3D model, and each grid point is encoded with at least distance information of the grid point from at least one functional surface. The distance information may comprise a distance value, e.g. an Euclidean distance of the grid point to a closest point on the at least one functional surface and/or a color distance of the grid point to a closest point on the at least one functional surface. Therefore, in one example the encoded representation of each discretized 3D model comprises a distance field representation of the corresponding discretized 3D model. In another example, the distance information comprises a "+" or "-" sign, depending on the location of the grid point relative to the at least one functional surface, whereby grid points which are located outside the at least one functional surface are encoded with a "+" sign, and grid points which are located inside the at least one functional surface are encoded with a "-" sign. In this example, the encoded representation of the discretized 3D model of each restoration case may comprise a binary representation. The at least one functional surface may comprise one or more of a surface of a preparation jaw, a surface of an antagonist jaw, and/or a minimal surface boundary. The surface of the preparation jaw, the surface of the antagonist jaw and/or the minimal surface boundary may be determined for each restoration case in 701 based on the corresponding 3D surface data 702, or they may be retrieved from the corresponding metadata 704.

In step 707, a discretized representation of the designed dental restoration(s) 703 is generated for each restoration case in 701, based on the corresponding discretized 3D model of the same restoration case. For example, if the discretized 3D model of a given restoration case comprises a discretization grid centered in a centroid or center of mass of the preparation surface and having a predefined size, the corresponding designed dental restoration(s) 703 is discretized by using the same discretization grid, having the same center, resolution, and size. For example, the discretized 3D model may comprises a structured discretization grid with a size of 138 × 64 × 64 voxels, having a sample rate of 1 grid point over a box covering 40 × 20 × 20 mm. The orientation of the grid may be such that the x-axis has 138 samples over 40mm and is placed along the teeth line, the y-axis has 64 samples over 20mm and is placed in the lingual buccal dimension, and the z-axis has 64 samples over 20mm and points in the occlusal direction. Accordingly, the corresponding designed dental restoration(s) 703 is discretized in step 707 using a grid with the size of 138 × 64 × 64 voxels, having a sample rate of 1 grid point over a box covering 40 × 20 × 20 mm, wherein the x-axis has 138 samples over 40mm and is placed along the teeth line, the y-axis has 64 samples over 20mm and is placed in the lingual buccal dimension, and the z-axis has 64 samples over 20mm and points in the occlusal direction. In other words, the same discretization grid may be used to discretize both the at least a part of the 3D surface data 702 and the corresponding designed dental restoration(s) 703 for each restoration case in 701.

In step 708, an encoded representation of the discretized designed dental restoration(s) is generated. In particular, each point of the encoded representation of the discretized designed dental restoration(s) is encoded with at least distance information of the point to the outer surface of the corresponding designed dental restoration(s) 703. For each restoration case, the encoded representation of the discretized designed dental restoration(s) may be of the same type as the encoded representation of the corresponding discretized 3D model. For example, if the encoded representation of the discretized 3D model is a distance field representation, then the corresponding encoded representation of the discretized designed dental restoration(s) is a distance field. In another example, the encoded representation of the discretized 3D model may be a truncated distance field, and the corresponding encoded representation of the discretized designed dental restoration(s) is a truncated distance field. In yet another example, the encoded representation of the discretized 3D model may be a binary representation, and the corresponding encoded representation of the discretized designed dental restoration(s) is a binary representation.

In step 709, the model is trained by inputting into the model the encoded representation of the discretized 3D model of each restoration case generated in step 706, the encoded representation of the discretized designed dental restoration(s) of each restoration case generated in step 708, and the metadata 704 of each restoration case. In some examples, the trained model may be a distance field regression trained neural network. The trained neural network may be a Deep Convolutional Neural Network, whose structure may enable to generate an output having the same resolution as the corresponding input. For example, the training methodology of the trained neural network may be a forward and backwards propagation using a gradient descent optimizer.

The result of the training in step 709 is a trained neural network which is configured to receive, as an input, an encoded representation, e.g. a distance field representation, of at least a part of 3D surface data comprising a preparation surface, and to output an encoded representation of a prediction of at least a part of a dental restoration. The at least a part of the dental restoration may be an outer surface of the dental restoration, or it may be an outer surface and an inner structure of the dental restoration.

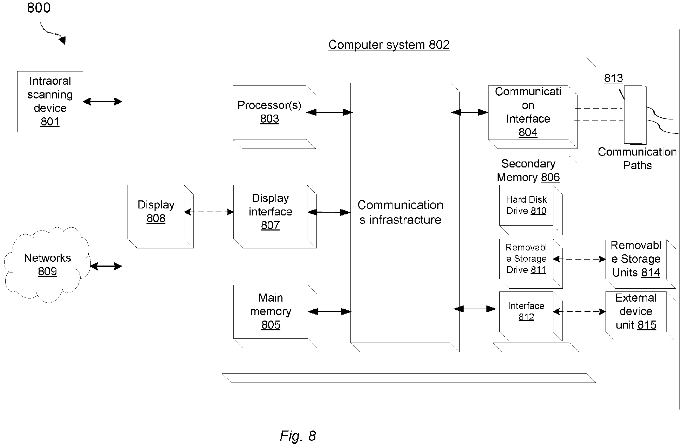

Fig. 8 schematically illustrates a system 800 according to the present disclosure. The system 800 may comprise a plurality of components enabling data processing, storage, communication and user interaction. The computer system described herein may comprise an intraoral scanning device 801, and a computer system 802 comprising one or more processor(s) 803, a communication interface 804, a main memory 805, a secondary memory 806, a display interface 807, and a display 808. The one or more processor(s) 803 may be configured to execute one or more steps of the computer-implemented method disclosed herein. The communication interface 804 may be configured to support a number of communication paths 813, such as Ethernet, Wi-fi, Bluetooth etc, for enabling the computer system 802 to access a number of communication paths. For example, the communication interface 804 may enable the computer system 802 to exchange data with the intraoral scanning device 801, e.g. the communication interface 804 may enable the computer system 802 to receive digital 3D surface data of a dentition from the intraoral scanning device 801. The communication interface 804 may be further configured to enable the computer system 802 to receive and/or exchange data and to communicate with external devices and networks, such as one or more cloud networks 809. One or more steps of the computer-implemented discloser herein may be executed on the one or more cloud networks 809. For example, the cloud networks 809 may comprise executable instructions to process one or more encoded representations, e.g. one or more distance fields according to the present disclosure, by means of a trained model, e.g. a model trained according to a training method as the one illustrated as reference number 700 in Figure 7. The communication interface 804 may be further configured to receive one or more input signal(s) from other devices, which causes the computer to execute one or more steps of the computer-implemented method disclosed herein.

The secondary memory 806 may comprise a hard disk drive 810, a removable storage drive 811 and an interface 812. The hard disk drive 810 may be configured to provide non-volatile storage for the computer system 802 for storing computer-readable programs, operating system files, user data, etc. The hard disk drive 810 may be further configured to provide persistent storage for the computer system 802 for retaining data even when the system is powered off. The removable storage drive 811 may be configured to enable the computer to access removable storage units 814 for reading from and/or writing to the removable storage units. For example, the removable storage drive 811 may be a CD/DVD drive or USB port. The interface 812 may be configured to connect various external device units 815, such as keyboards, mice, printers, scanners, or audio devices, to the computer system.

Although some embodiments have been described and shown in detail, the disclosure is not restricted to such details, but may also be embodied in other ways within the scope of the subject matter defined in the following claims. In particular, it is to be understood that other embodiments may be utilized, and structural and functional modifications may be made without departing from the scope of the present invention.

A claim may refer to any of the preceding claims, and "any" is understood to mean "any one or more" of the preceding claims. As used, the singular forms "a," "an," and "the" are intended to include the plural forms as well (i.e. to have the meaning "at least one"), unless expressly stated otherwise. It will be further understood that the terms "includes," "comprises," "including," and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items. The steps of any disclosed method is not limited to the exact order stated herein, unless expressly stated otherwise.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

The claims are not intended to be limited to the aspects shown herein, but is to be accorded the full scope consistent with the language of the claims, wherein reference to an element in the singular is not intended to mean "one and only one" unless specifically so stated, but rather "one or more." Unless specifically stated otherwise, the term "some" refers to one or more.

### Further details

Embodiments of the invention are disclosed in the following items.
1. A computer-implemented method for automatically designing a dental restoration for placing in a dentition of a patient, the method comprising the steps of:
   - obtaining digital three-dimensional (3D) surface data representing a surface of the dentition of the patient in 3D space, wherein the surface comprises a preparation surface configured to receive the dental restoration;
   - pre-processing the 3D surface data to generate a discretized 3D model comprising granularity elements defining a discretized representation of at least a part of the 3D surface data, wherein the at least a part of the 3D surface data comprises the preparation surface;
   - pre-processing the discretized 3D model to generate an encoded representation of the discretized 3D model, wherein the encoded representation comprises the granularity elements, and each granularity element is encoded with at least distance information from the point to at least one functional surface;
   - processing the encoded representation by using a trained model configured to process the encoded representation;
   - receiving an output of the processing from the trained model, wherein the output comprises at least a part of the dental restoration; and
   - automatically designing the dental restoration based on the received output.
2. The computer-implemented method according to item 1, wherein the 3D surface data comprises data representing a surface of an upper jaw of the dentition and data representing a surface of a lower jaw of the dentition.
3. The computer-implemented method according to item 2, further comprising obtaining 3D surface data of the upper jaw in occlusion with the lower jaw, thereby obtaining information of a relative alignment between the upper jaw and the lower jaw.
4. The computer-implemented method according to any of the preceding items, wherein the granularity elements comprise a plurality of facets of a mesh.
5. The computer-implemented method according to any of items 1-3, wherein the granularity elements comprise a point cloud, and wherein the point cloud comprises points in 3D space.
6. The computer-implemented method according to any of the preceding items, further comprising determining or retrieving one or more structural features of the preparation surface, wherein the one or more structural features comprise one or more of a location of the preparation surface, and/or a margin line of the preparation surface.
7. The computer-implemented method according to any of the preceding items, further comprising determining or retrieving one or more design constraints to be enforced when designing the dental restoration, wherein the one or more design constraints comprise one or more of a determined insertion direction of the dental restoration, a planned manufacturing material of the dental restoration and/or a determined inner surface of the dental restoration.
8. The computer-implemented method according to item 7, wherein a minimal thickness of the dental restoration to be enforced when designing the dental restoration is determined based on the planned manufacturing material of the dental restoration.
9. The computer-implemented method according to any of the preceding items, wherein an input of the trained model further comprises one or more of a relative alignment between an upper jaw and a lower jaw of the dentition, one or more structural features of the preparation surface, and/or one or more design constraints to be enforced when designing the dental restoration.
10. The computer-implemented method according to any of the preceding items, wherein the discretized 3D model comprises a discretization grid.
11. The computer-implemented method according to item 10, wherein the discretization grid is a structured discretization grid encompassing at least the preparation surface and the discretization grid having a desired size.
12. The computer-implemented method according to item 11, wherein the desired size of the structured discretization grid is based on a pose of the preparation surface.
13. The computer-implemented method according to any of items 11 or 12, wherein a position of the preparation surface within the structured discretization grid is based on the pose of the preparation surface.
14. The computer-implemented method according to any of items 12-13, wherein the pose of the preparation surface is determined based on the obtained 3D surface data.
15. The computer-implemented method according to any of items 12-13, wherein the pose of the preparation surface is retrieved based on a received input.
16. The computer-implemented method according to item 11, wherein the desired size of the structured discretization grid is based on a desired resolution of the output of the trained model.
17. The computer-implemented method according to item 11, wherein the structured discretization grid entirely encompasses the obtained 3D surface data, whereby the discretized 3D model is a discretized representation of the obtained 3D surface data.
18. The computer-implemented method according to any of items 11-17, wherein the desired size of the structured discretization grid is a predefined feature of the method or the desired size of the structured discretization grid is selected by a user.
19. The computer-implemented method according to any of items 11-18, wherein the structured discretization grid comprises an x-axis, a y-axis, and a z-axis, and wherein the x-axis is oriented along a tooth line, the y-axis is oriented along a lingual dimension or along a buccal dimension, and the z-axis is oriented along an occlusal direction.
20. The computer-implemented method according to any of the preceding items, wherein pre-processing the discretized 3D model comprises:
   - determining, for each granularity element of the discretized 3D model, at least one distance value, wherein the at least one distance value quantifies a distance of the granularity element to the at least one functional surface; and
   - encoding each granularity element of the discretized 3D model with the corresponding at least one distance value to generate the encoded representation.
21. The computer-implemented method according to any of the preceding items, wherein the at least one functional surface comprises one or more of a surface of a preparation jaw of the dental restoration, a surface of an antagonist jaw of the dental restoration, and/or a minimal surface boundary of the dental restoration.
22. The computer-implemented method according to item 21, further comprising retrieving a planned manufacturing material for use in a manufacturing process of the dental restoration, and determining the minimal surface boundary based on the planned manufacturing material.
23. The computer-implemented method according to any of items 21-22, wherein the minimal surface boundary defines a design constraint to be enforced when designing the dental restoration, the design constraint comprising a minimal outer surface required to design the dental restoration with one or more planned functional properties.
24. The computer-implemented method according to item 23, wherein the one or more planned functional properties comprise a mechanical strength.
25. The computer-implemented method according to any of the preceding items, wherein the encoded representation is a distance field representation.
26. The computer-implemented method according to any of the preceding items, wherein the trained model is a trained neural network.
27. The computer-implemented method according to item 26, wherein the trained neural network is a Deep Convolutional Neural Network.
28. The computer-implemented method according to any of the preceding items, wherein a resolution of the input of the trained model is the same as a resolution of the output of the trained model.
29. The computer-implemented method according to any of the preceding items, wherein the dental restoration comprises one or more of a crown, a veneer, an inlay, an onlay, a dental bridge, a denture, and/or a removable partial denture.
30. The computer-implemented method according to any of the preceding items, wherein the output of the trained model comprises a distance field representing at least a part of an outer surface of the dental restoration.
31. The computer-implemented method according to item 30, wherein automatically designing the dental restoration comprises converting the output distance field to a mesh model.
32. The computer-implemented method according to any of the preceding items, wherein automatically designing the dental restoration further comprises retrieving a digital template tooth model and morphing the output of the trained model with the digital template tooth model.
33. The computer-implemented method according to item 32, wherein the morphing comprises adapting the digital template tooth model to the output of the trained model to refine one or more anatomical features of the dental restoration, the one or more anatomical features comprising ridges and grooves.
34. The computer-implemented method according to any of the preceding items, wherein the dental restoration is a partial restoration.
35. The computer-implemented method according to item 34, wherein the partial restoration comprises a veneer or an inlay.
36. The computer-implemented method according to any of items 34-35, further comprising combining the output of the trained model with at least a part of the surface comprised in the obtained 3D surface data to automatically design the partial dental restoration.
37. The computer-implemented method according to item 36, wherein the at least a part of the surface comprises a tooth surface which is intact after a preparation process of the tooth, and wherein the tooth is configured to receive the dental restoration.
38. The computer-implemented method according to item 36, wherein the at least a part of the surface comprises a tooth surface before the tooth suffered any damage, and wherein the tooth is configured to receive the dental restoration.
39. The computer-implemented method according to any of the preceding items, further comprising iteratively repeating the method of any of one or more of items 1-38 at least a number *N* of times to design a number *N* of dental restorations.
40. The computer-implemented method according to item 39, wherein the at least a number *N* of times is based on a convergence criterion.
41. The computer-implemented method according to any of items 39-40, wherein the number *N* of dental restorations are connected to form a dental bridge.
42. The computer-implemented method according to any of the preceding items, further comprising determining a certainty score of the output of the trained model.
43. The computer-implemented method according to any of the preceding items, wherein the output of the trained model comprises an outer surface of the dental restoration.
44. The computer-implemented method according to any of the preceding items, wherein the output of the trained model comprises an inner structure of the dental restoration.
45. A computer program product comprising instructions which, when executed by a computer, cause the computer to perform the method according to any of the preceding items.
46. A non-volatile computer-readable medium comprising instructions which, when executed by a computer, cause the computer to perform the method according to any of the preceding items.
47. A system comprising:
   ∘ an intraoral scanning device configured to acquire digital three-dimensional (3D) surface data of a dentition of a patient; and
   ∘ a computer system comprising:
      - a display;
      - a communication interface;
      - a processing unit;
      - a memory unit containing a program content executable by the processing unit, the program content comprising executable instructions to:
         a. obtain 3D surface data of the dentition of the patient representing a surface of the dentition in three-dimensional (3D) space, wherein the surface comprises a preparation surface configured to receive the dental restoration;
         b. pre-process the obtained 3D surface data to generate a discretized 3D model comprising granularity elements defining a discretized representation of at least a part of the 3D surface data, wherein the at least a part of the 3D surface data comprises the preparation surface;
         c. pre-process the discretized 3D model to generate an encoded representation of the discretized 3D model, wherein the encoded representation comprises the granularity elements, and each granularity element is encoded with at least distance information from the point to at least one functional surface;
         d. process the encoded representation by using a trained model configured to process the encoded representation;
         e. receive an output of the processing from the trained model, wherein the output comprises at least a part of the dental restoration; and
         f. automatically design the dental restoration based on the output of the trained model.
48. The system according to item 47, wherein the communication interface is configured to enable the computer system to exchange data with one or more external devices and with one or more external networks.
49. The system according to item 48, wherein the one or more external networks comprise at least one cloud network.
50. The system according to item 49, wherein the at least one cloud network comprises executable instructions to process the encoded representation using the trained model.
51. The system according to any of items 47-50, further comprising a Computer Aid Manufacturing (CAM) device configured to manufacture at least a part of the digitally designed dental restoration.
52. The system according to any of items 47-51, further comprising an additive manufacturing device configured to manufacture at least a part of the digitally designed dental restoration.
53. The system according to item 52, wherein the additive manufacturing device is a 3D printer.

## Claims

1. A computer-implemented method for automatically designing a dental restoration for placing in a dentition of a patient, the method comprising the steps of:
- obtaining digital three-dimensional (3D) surface data representing a surface of the dentition of the patient in 3D space, wherein the surface comprises a preparation surface configured to receive the dental restoration;
- pre-processing the 3D surface data to generate a discretized 3D model comprising granularity elements defining a discretized representation of at least a part of the 3D surface data, wherein the at least a part of the 3D surface data comprises the preparation surface;
- pre-processing the discretized 3D model to generate an encoded representation of the discretized 3D model, wherein the encoded representation comprises the granularity elements, and each granularity element is encoded with at least distance information from the point to at least one functional surface;
- processing the encoded representation by using a trained model configured to process the encoded representation;
- receiving an output of the processing from the trained model, wherein the output comprises at least a part of the dental restoration; and
automatically designing the dental restoration based on the received output.

2. The computer-implemented method according to claim 1, wherein the granularity elements comprise a point cloud, and wherein the point cloud comprises points in 3D space.

3. The computer-implemented method according to any of the preceding claims, further comprising determining or retrieving one or more structural features of the preparation surface, wherein the one or more structural features comprise one or more of a location of the preparation surface, and/or a margin line of the preparation surface.

4. The computer-implemented method according to any of the preceding claims, further comprising determining or retrieving one or more design constraints to be enforced when designing the dental restoration, wherein the one or more design constraints comprise one or more of a determined insertion direction of the dental restoration, a planned manufacturing material of the dental restoration and/or a determined inner surface of the dental restoration.

5. The computer-implemented method according to any of the preceding claims, wherein an input of the trained model further comprises one or more of a relative alignment between an upper jaw and a lower jaw of the dentition, one or more structural features of the preparation surface, and/or one or more design constraints to be enforced when designing the dental restoration.

6. The computer-implemented method according to any of the preceding claims, wherein the discretized 3D model comprises a discretization grid.

7. The computer-implemented method according to claim 6, wherein the discretization grid is a structured discretization grid encompassing at least the preparation surface and the discretization grid having a desired size.

8. The computer-implemented method according to claim 7, wherein a position of the preparation surface within the structured discretization grid is based on a pose of the preparation surface.

9. The computer-implemented method according to any of the preceding claims, wherein the at least one functional surface comprises one or more of a surface of a preparation jaw of the dental restoration, a surface of an antagonist jaw of the dental restoration, and/or a minimal surface boundary of the dental restoration.

10. The computer-implemented method according to any of the preceding claims, wherein the encoded representation is a distance field representation.

11. The computer-implemented method according to any of the preceding claims, wherein the output of the trained model comprises a distance field representing at least a part of an outer surface of the dental restoration.

12. The computer-implemented method according to any of the preceding claims, wherein automatically designing the dental restoration further comprises retrieving a digital template tooth model and morphing the output of the trained model with the digital template tooth model.

13. The computer-implemented method according to any of the preceding claims, wherein the dental restoration is a partial dental restoration, and wherein the method further comprises combining the output of the trained model with at least a part of the surface comprised in the obtained 3D surface data to automatically design the partial dental restoration.

14. The computer-implemented method according to claim 13, wherein the at least a part of the surface comprises a tooth surface which is intact after a preparation process of the tooth, and wherein the tooth is configured to receive the dental restoration.

15. A system comprising:
∘ an intraoral scanning device configured to acquire digital three-dimensional (3D) surface data of a dentition of a patient; and
∘ a computer system comprising:
- a display;
- a communication interface;
- a processing unit;
- a memory unit containing a program content executable by the processing unit, the program content comprising executable instructions to:
g. obtain 3D surface data of the dentition of the patient representing a surface of the dentition in three-dimensional (3D) space, wherein the surface comprises a preparation surface configured to receive the dental restoration;
h. pre-process the obtained 3D surface data to generate a discretized 3D model comprising granularity elements defining a discretized representation of at least a part of the 3D surface data, wherein the at least a part of the 3D surface data comprises the preparation surface;
i. pre-process the discretized 3D model to generate an encoded representation of the discretized 3D model, wherein the encoded representation comprises the granularity elements, and each granularity element is encoded with at least distance information from the point to at least one functional surface;
j. process the encoded representation by using a trained model configured to process the encoded representation;
k. receive an output of the processing from the trained model, wherein the output comprises at least a part of the dental restoration; and
l. automatically design the dental restoration based on the output of the trained model.
